# EUROPEAN PATENT APPLICATION

(11) **EP 2 746 273 A1**
(43) Date of publication of application: **25.06.2014**
(21) Application number: 12824146.0
(22) Date of filing: 15.08.2012
(51) Int. Cl.: C07D 403/14, H01L 51/50

(54) **BISCARBAZOLE DERIVATIVE AND ORGANIC ELECTROLUMINESCENCE ELEMENT USING SAME**

(30) Priority: 18.08.2011 JP 2011179246
(71) Applicant: Idemitsu Kosan Co., Ltd, Tokyo 100-8321 (JP)
(72) Inventor: INOUE Tetsuya, Sodegaura-shi Chiba 299-0293 (JP); ITO Mitsunori, Sodegaura-shi Chiba 299-0293 (JP); NISHIMURA Kazuki, Sodegaura-shi Chiba 299-0293 (JP); HIBINO Kumiko, Sodegaura-shi Chiba 299-0293 (JP)
(74) Representative: Gille Hrabal
(86) International application number: PCT/JP2012/070754
(87) International publication number: WO 2013/024872

(57) **Abstract**

A biscarbazole derivative is represented by the following formula (1). A₁ and A₂ of the following formula (1) represent an aromatic hydrocarbon group having 6 to 30 ring carbon atoms or an aromatic heterocyclic group having 1 to 30 ring carbon atoms. However, at least one of A₁ and A₂ represents an aromatic heterocyclic group having 1 to 30 ring carbon atoms. Y₁ to Y₁₅ represent CR or a nitrogen atom. One of Y₈ to Y₁₁ is C (carbon atom) obtained by removing R from CR. The obtained C is bonded to L₃. R each independently represents a hydrogen atom, an aromatic hydrocarbon group or the like. L₁ to L₃ represent a single bond or a divalent linking group. When L₃ is a single bond and is bonded to Y₁₁, L₁ and L₂ are divalent linking groups.

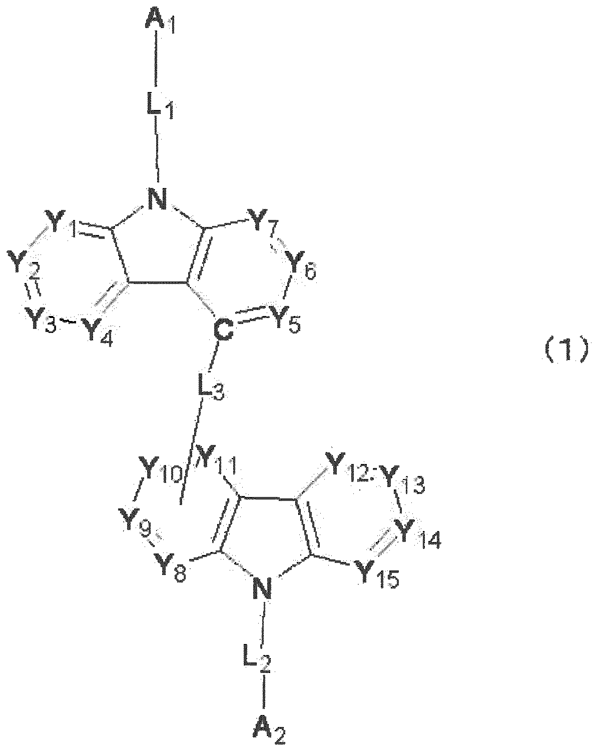

## Description

### TECHNICAL FIELD

The present invention relates to a biscarbazole derivative and an organic electroluminescence device using the biscarbazole derivative.

### BACKGROUND ART

When voltage is applied to an organic electroluminescence device (hereinafter, occasionally abbreviated as an organic EL device), holes are injected from an anode into an emitting layer while electrons are injected from a cathode into the emitting layer. In the emitting layer, the injected holes and electrons are recombined to form excitons. According to the electron spin statistics theory, singlet excitons and triplet excitons are generated at a ratio of 25%:75%. In a classification by the emission principle, in a fluorescent organic EL device which uses emission caused by singlet excitons, an internal quantum efficiency is believed to be 25% at the maximum. On the other hand, in a phosphorescent organic EL device which uses emission caused by triplet excitons, it has been known that the internal quantum efficiency can be improved up to 100% when intersystem crossing from the singlet excitons occurs efficiently.

In a typical organic EL device, the most suitable device design has been made depending on fluorescent and phosphorescent emission mechanism. Particularly, it has been known that a simple application of a fluorescent device technique for designing the phosphorescent organic EL device cannot provide a highly efficient phosphorescent organic EL device because of a luminescence property of the phosphorescent organic EL device. The reasons are generally considered as follows.

First of all, since the phosphorescent emission is generated using triplet excitons, an energy gap of a compound used for the emitting layer must be large. This is because a value of an energy gap (hereinafter, also referred to as singlet energy) of a compound is typically larger than a value of triplet energy (i.e., an energy gap between energy in the lowest triplet state and energy in the ground state) of the compound.

Accordingly, in order to efficiently confine triplet energy of a phosphorescent dopant material within the device, a host material having a larger triplet energy than the phosphorescent dopant material must be used in the emitting layer. Moreover, an electron transporting layer and a hole transporting layer must be provided adjacent to the emitting layer. In the electron transporting layer and the hole transporting layer, a compound having a larger triplet energy than the phosphorescent dopant material must be used. Thus, according to the designing idea of the typical organic EL device, a compound having a larger energy gap than a compound used in the fluorescent organic EL device is used in the phosphorescent organic EL device, resulting in increasing a drive voltage of the entire organic EL device.

Since a hydrocarbon compound useful for the fluorescent device and exhibiting a high oxidation resistance and a high reduction resistance has a broad π electron cloud, an energy gap of the hydrocarbon compound is small. For this reason, such a hydrocarbon compound is unlikely to be selected for the phosphorescent organic EL device, but an organic compound containing a hetero atom such as oxygen and nitrogen is selected. As a result, a lifetime of the phosphorescent organic EL device is adversely shorter than that of the fluorescent organic EL device.

Moreover, a device performance of the phosphorescent organic EL device is greatly affected by an exciton relaxation rate of triplet excitons much longer than that of singlet excitons in the phosphorescent dopant material. In other words, with respect to emission from the singlet excitons, since a relaxation rate leading to emission is so fast that the singlet excitons are unlikely to diffuse to the neighboring layers of the emitting layer (e.g., the hole transporting layer and the electron transporting layer), efficient emission is expected. On the other hand, since emission from the triplet excitons is based on the forbidden spin transition and exhibits a slow relaxation rate, the triplet excitons are likely to diffuse to the neighboring layers, so that the triplet excitons are thermally energy-deactivated from a compound other than a specific phosphorescent compound. In short, in the phosphorescent organic EL device, control of the recombination region of the electrons and the holes is more important than in the fluorescent organic EL device.

For the above reasons, advancement of the phosphorescent organic EL device requires material selection and device design different from those of the fluorescent organic EL device.

Technique of using a carbazole derivative, aromatic amine derivative, quinolynol metal complex and the like as a host material (phosphorescent host material) to be combined with the above phosphorescent dopant material is disclosed. However, none of the above examples of the host material exhibits sufficient luminous efficiency and low drive voltage

Recently, technique of using a biscarbazole derivative as the phosphorescent host material in place of the above examples of the phosphorescent host material has been disclosed (see, for instance, Patent Literatures 1 to 3).

### CITATION LIST

### PATENT LITERATURES

Patent Literature 1: Japanese Patent No. 4357781
Patent Literature 2: JP-A-2008-135498
Patent Literature 3: International Publication No. WO2011/019156

### SUMMARY OF THE INVENTION

### PROBLEMS TO BE SOLVED BY THE INVENTION

Patent literatures 1 to 3 disclose an organic EL device using as a phosphorescent host material a biscarbazole derivative in which carbazolyl groups are bonded to each other at their positions 3.

An object of the invention is to provide a biscarbazole derivative capable of confining excitation energy for phosphorescence in an emitting layer of an organic EL device more effectively as compared with typical compounds, and an organic EL device using the biscarbazole derivative.

### MEANS FOR SOLVING THE PROBLEMS

After conducting concentrated studies in order to achieve the above object, the inventors have found that a biscarbazole derivative, in which one carbazolyl group is bonded at its position 4 to any position other than the N-position (position 9) of the other carbazolyl group, tends to exhibit a larger triplet energy than a compound in which carbazolyl groups are bonded to each other at their positions 3.

The inventors have achieved the invention on the above findings.

A biscarbazole derivative according to an aspect of the invention is represented by a formula (1) below.

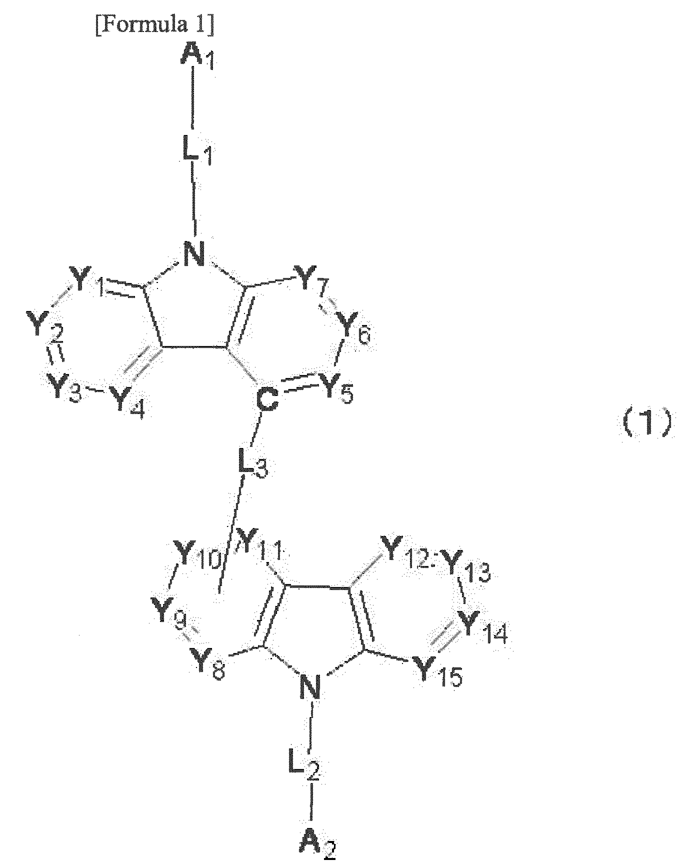

In the formula (1), A₁ and A₂ represent a substituted or unsubstituted aromatic hydrocarbon group having 6 to 30 ring carbon atoms, or a substituted or unsubstituted aromatic heterocyclic group having 1 to 30 ring carbon atoms, with the proviso that at least one of A₁ and A₂ represents a substituted or unsubstituted aromatic heterocyclic group having 1 to 30 ring carbon atoms.

Y₁ to Y₄ each are a nitrogen atom or a carbon atom to be bonded to the following R, with the proviso that, when adjacent two of Y₁ to Y₄ are carbon atoms, a ring including the adjacent carbon atoms may be formed without the adjacent carbon atoms being bonded to R.

Y₅ to Y₇ are a nitrogen atom or a carbon atom to be bonded to the following R, with the proviso that, when adjacent two of Y₅ to Y₇ are carbon atoms, a ring including the adjacent carbon atoms may be formed without the adjacent carbon atoms being bonded to R.

One of Y₈ to Y₁₁ is a carbon atom bonded to L₃. Except for the one of Y₈ to Y₁₁ that is bonded to L₃, Y₈ to Y₁₁ are a nitrogen atom or a carbon atom to be bonded to the following R, with the proviso that, when adjacent two of Y₈ to Y₁₁ are carbon atoms, a ring including the adjacent carbon atoms may be formed without the adjacent carbon atoms being bonded to R.

Y₁₂ to Y₁₅ are a nitrogen atom or a carbon atom to be bonded to the following R, with the proviso that, when adjacent two of Y₁₂ to Y₁₅ are carbon atoms, a ring including the adjacent carbon atoms may be formed without the adjacent carbon atoms being bonded to R.

Herein, Y₁ to Y₁₅ will be described in detail. When adjacent two of Y₁ to Y₁₅, for instance, Y₁ and Y₂ are carbon atoms and form a ring, Y₁ and Y₂ are not bonded to R and another ring structure including Y₁ and Y₂ may be formed in addition to a six-membered ring of a biscarbazole skeleton including Y₁ to Y₄. When Y₁ and Y₂ form the ring structure, Y₃ and Y₄ are a carbon atom or a nitrogen atom. When Y₃ and Y₄ are carbon atoms, Y₃ and Y₄ are not bonded to R and another ring structure including Y₃ and Y₄ may be formed in addition to a six-membered ring of a biscarbazole skeleton including Y₁ to Y₄. The same applies to Y₅ to Y₇, Y₈ to Y₁₁, and Y₁₂ to Y₁₅.

In the formula (1), R independently represents a hydrogen atom, a substituted or unsubstituted aromatic hydrocarbon group having 6 to 30 ring carbon atoms, a substituted or unsubstituted aromatic heterocyclic group having 1 to 30 ring carbon atoms, a substituted or unsubstituted linear, branched or cyclic alkyl group having 1 to 30 carbon atoms, a substituted or unsubstituted alkoxy group having 1 to 30 carbon atoms, a substituted or unsubstituted aryloxy group having 6 to 30 ring carbon atoms, a substituted or unsubstituted aralkyl group having 7 to 30 carbon atoms, a substituted or unsubstituted haloalkyl group having 1 to 30 carbon atoms, a substituted or unsubstituted haloalkoxy group having 1 to 30 carbon atoms, a substituted or unsubstituted trialkylsilyl group having 3 to 30 carbon atoms, a substituted or unsubstituted dialkylarylsilyl group having 8 to 40 carbon atoms, a substituted or unsubstituted alkyldiarylsilyl group having 13 to 50 carbon atoms, a substituted or unsubstituted triarylsilyl group having 18 to 60 carbon atoms, a halogen atom, a cyano group, a hydroxyl group, a nitro group, or a carboxy group.

L₁ to L₃ represent a single bond or a divalent linking group, with the proviso that, when L₃ is a single bond and is bonded to Y₁₁, L₁ and L₂ are divalent linking groups.

In the biscarbazole derivative according to the aspect of the invention, the biscarbazole derivative represented by the formula (1) is preferably represented by the following formula (2), (3) or (4).

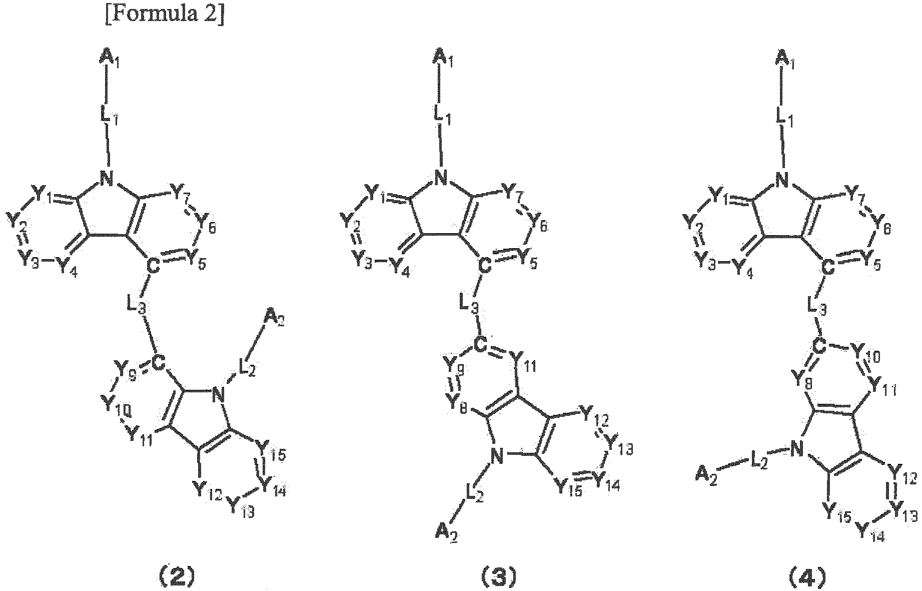

In the above formulae (2) to (4), A₁, A₂, Y₁ to Y₁₅ and L₁ to L₃ are the same as A₁, A₂, Y₁ to Y₁₅ and L₁ to L₃ in the formula (1).

In the biscarbazole derivative according to the aspect of the invention, the biscarbazole derivative represented by the formula (1) is preferably represented by the following formula (3) or (4).

In the biscarbazole derivative according to the aspect of the invention, L₃ in the formula (1) is preferably a single bond.

In the biscarbazole derivative according to the aspect of the invention, the aromatic heterocyclic group is preferably a nitrogen-containing aromatic heterocyclic group.

In the biscarbazole derivative according to the aspect of the invention, the nitrogen-containing aromatic heterocyclic group is preferably a heterocyclic group having a pyrimidine skeleton or a heterocyclic group having a triazine skeleton.

In the biscarbazole derivative according to the aspect of the invention, at least one of L₁ and L₂ in the formula (1) is preferably a divalent group derived from a substituted or unsubstituted aromatic hydrocarbon compound having 6 to 30 ring carbon atoms, or a divalent group derived from a substituted or unsubstituted aromatic heterocyclic compound having 1 to 30 ring carbon atoms.

An organic electroluminescence device according to another aspect of the invention includes: a cathode; an anode; and an organic compound layer between the cathode and the anode, in which the organic compound layer includes any one of the biscarbazole derivatives according to the above aspects of the invention.

In the organic electroluminescence device according to the above aspect of the invention, it is preferable that the organic compound layer includes a plurality of organic thin-film layers including an emitting layer, in which at least one of the plurality of organic thin-film layers includes any one of the carbazole derivatives according to the above aspects of the invention.

In the organic electroluminescence device according to the above aspect of the invention, the emitting layer preferably includes any one of the biscarbazole derivatives according to the above aspects of the invention.

In the organic electroluminescence device according to the above aspect of the invention, the emitting layer preferably includes a phosphorescent material.

In the organic electroluminescence device according to the above aspect of the invention, the phosphorescent material preferably includes an ortho-metalated complex of a metal atom selected from iridium (Ir), osmium (Os) and platinum (Pt).

In the organic electroluminescence device according to the above aspect of the invention, the emitting layer further preferably includes an aromatic amine derivative.

In the organic electroluminescence device according to the above aspect of the invention, the plurality of organic thin-film layers preferably include a hole transporting layer, the emitting layer and an electron transporting layer.

By using the biscarbazole derivative according to the above aspects of the invention in an organic EL device, a luminous efficiency is improvable.

### BRIEF DESCRIPTION OF DRAWINGS

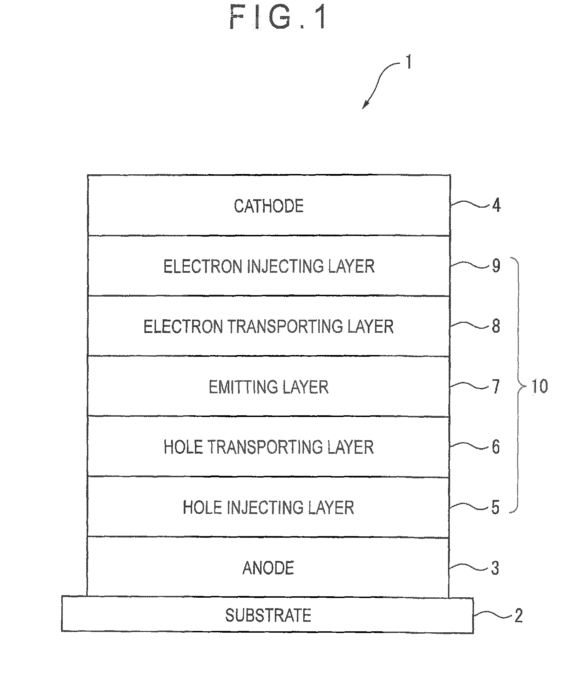

Fig. 1 schematically shows an exemplary arrangement of an organic electroluminescence device according to an exemplary embodiment of the invention.

### DESCRIPTION OF EMBODIMENT(S)

The invention will be described below in detail.

### Biscarbazole Derivative

A biscarbazole derivative according to an exemplary embodiment of the invention is represented by the above formula (1).

The biscarbazole derivative according to the exemplary embodiment has a structure in which one carbazolyl group is bonded at its position 4 to any position other than the N-position (position 9) of the other carbazolyl group as shown in the formula (1). Since the biscarbazole derivative according to the exemplary embodiment has such a structure, conjugation is cut and an energy gap is increased.

Accordingly, the biscarbazole derivative according to the exemplary embodiment exhibits a larger triplet energy than the biscarbazole derivative in which the carbazolyl groups are bonded to each other at their positions 3.

In the biscarbazole derivative according to the exemplary embodiment, as shown in the formula (1), at least one of A₁ and A₂ that are bonded to the N-position (position 9) of the carbazolyl group directly or through linking groups L₁ to L₂ is a substituted or unsubstituted aromatic heterocyclic group having 1 to 30 ring carbon atoms. With such a structure, a HOMO and a LUMO of the biscarbazole derivative according to the exemplary embodiment can clearly be separated as compared with a structure in which the aromatic heterocyclic group is bonded to a benzene ring of the carbazolyl group. Accordingly, the biscarbazole derivative according to the exemplary embodiment is expected to exhibit an excellent resistance to holes and electrons.

Examples of the aromatic hydrocarbon group having 6 to 30 ring carbon atoms in the formula (1), including a fused aromatic hydrocarbon group, are a phenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 4'-methylbiphenylyl group, 4"-t-butyl-p-terphenyl-4-yl group, o-cumenyl group, m-cumenyl group, p-cumenyl group, 2,3-xylyl group, 3,4-xylyl group, 2,5-xylyl group, mesityl group, m-quarter-phenyl group, 1-naphthyl group, 2-naphthyl group, 1-phenanthrenyl group, 2-phenanthrenyl group, 3-phenanthrenyl group, 4-phenanthrenyl group, 9-phenanthrenyl group, 1-triphenylenyl group, 2-triphenylenyl group, 3-triphenylenyl group, 4-triphenylenyl group, 1-chrysenyl group, 2-chrysenyl group, 3-chrysenyl group, 4-chrysenyl group, 5-chrysenyl group, 6-chrysenyl group, 3-fluoranthenyl group, 4-fluoranthenyl group, 8-fluoranthenyl group, and 9-fluoranthenyl group.

Examples of the aromatic heterocyclic group having 1 to 30 ring carbon atoms in the formula (1), including a fused aromatic heterocyclic group, are a pyroryl group, pyrazinyl group, pyridinyl group, indolyl group, isoindolyl group, furyl group, benzofuranyl group, isobenzofuranyl group, dibenzofuranyl group, dibenzothiophenyl group, quinolyl group, isoquinolyl group, quinoxalinyl group, quinazolynyl group, carbazolyl group, phenanthrydinyl group, acridinyl group, phenanthrolinyl group, thienyl group, and a group formed from a pyridine ring, pyrazine ring, pyrimidine ring, pyridazine ring, triazine ring, indole ring, quinoline ring, acridine ring, pyrrolidine ring, dioxane ring, piperidine ring, morpholine ring, piperazine ring, carbazole ring, furan ring, thiophene ring, oxazole ring, oxadiazole ring, benzoxazole ring, thiazole ring, thiadiazole ring, benzothiazole ring, triazole ring, imidazole ring, benzimidazole ring, pyrane ring and dibenzofuran ring.

Further specifically, examples of the above group are a 1-pyroryl group, 2-pyroryl group, 3-pyroryl group, pyrazinyl group, 2-pyridinyl group, 2-pyrimidinyl group, 4-pyrimidinyl group, 5-pyrimidinyl group, 6-pyrimidinyl group, 1,2,3-triazine-4-yl group, 1,2,4-triazine-3-yl group, 1,3,5-triazine-2-yl group, 1-imidazolyl group, 2-imidazolyl group, 1-pyrazolyl group, 1-indolidinyl group, 2-indolidinyl group, 3-indolidinyl group, 5-indolidinyl group, 6-indolidinyl group, 7-indolidinyl group, 8-indolidinyl group, 2-imidazopyridinyl group, 3-imidazopyridinyl group, 5-imidazopyridinyl group, 6-imidazopyridinyl group, 7-imidazopyridinyl group, 8-imidazopyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 2-quinazolynyl group, 4-quinazolynyl group, 5-quinazolynyl group, 6-quinazolynyl group, 7-quinazolynyl group, 8-quinazolynyl group, 1-carbazolyl group, 2-carbazolyl group, 3-carbazolyl group, 4-carbazolyl group, 9-carbazolyl group, azacarbazolyl-1-yl group, azacarbazolyl-2-yl group, azacarbazolyl-3-yl group, azacarbazolyl-4-yl group, azacarbazolyl-5-yl group, azacarbazolyl-6-yl group, azacarbazolyl-7-yl group, azacarbazolyl-8-yl group, azacarbazolyl-9-yl group, 1-phenanthrydinyl group, 2-phenanthrydinyl group, 3-phenanthrydinyl group, 4-phenanthrydinyl group, 6-phenanthrydinyl group, 7-phenanthrydinyl group, 8-phenanthrydinyl group, 9-phenanthrydinyl group, 10-phenanthrydinyl group, 1-acridinyl group, 2-acridinyl group, 3-acridinyl group, 4-acridinyl group, 9-acridinyl group, 1,7-phenanthroline-2-yl group, 1,7-phenanthroline-3-yl group, 1,7-phenanthroline-4-yl group, 1,7-phenanthroline-5-yl group, 1,7-phenanthroline-6-yl group, 1,7-phenanthroline-8-yl group, 1,7-phenanthroline-9-yl group, 1,7-phenanthroline-10-yl group, 1,8-phenanthroline-2-yl group, 1,8-phenanthroline-3-yl group, 1,8-phenanthroline-4-yl group, 1,8-phenanthroline-5-yl group, 1,8-phenanthroline-6-yl group, 1,8-phenanthroline-7-yl group, 1,8-phenanthroline-9-yl group, 1,8-phenanthroline-10-yl group, 1,9-phenanthroline-2-yl group, 1,9-phenanthroline-3-yl group, 1,9-phenanthroline-4-yl group, 1,9-phenanthroline-5-yl group, 1,9-phenanthroline-6-yl group, 1,9-phenanthroline-7-yl group, 1,9-phenanthroline-8-yl group, 1,9-phenanthroline-10-yl group, 1,10-phenanthroline-2-yl group, 1,10-phenanthroline-3-yl group, 1,10-phenanthroline-4-yl group, 1,10-phenanthroline-5-yl group, 2,9-phenanthroline-1-yl group, 2,9-phenanthroline-3-yl group, 2,9-phenanthroline-4-yl group, 2,9-phenanthroline-5-yl group, 2,9-phenanthroline-6-yl group, 2,9-phenanthroline-7-yl group, 2,9-phenanthroline-8-yl group, 2,9-phenanthroline-10-yl group, 2,8-phenanthroline-1-yl group, 2,8-phenanthroline-3-yl group, 2,8-phenanthroline-4-yl group, 2,8-phenanthroline-5-yl group, 2,8-phenanthroline-6-yl group, 2,8-phenanthroline-7-yl group, 2,8-phenanthroline-9-yl group, 2,8-phenanthroline-10-yl group, 2,7-phenanthroline-1-yl group, 2,7-phenanthroline-3-yl group, 2,7-phenanthroline-4-yl group, 2,7-phenanthroline-5-yl group, 2,7-phenanthroline-6-yl group, 2,7-phenanthroline-8-yl group, 2,7-phenanthroline-9-yl group, 2,7-phenanthroline-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrole-1-yl group, 2-methylpyrrole-3-yl group, 2-methylpyrrole-4-yl group, 2-methylpyrrole-5-yl group, 3-methylpyrrole-1-yl group, 3-methylpyrrole-2-yl group, 3-methylpyrrole-4-yl group, 3-methylpyrrole-5-yl group, 2-t-butylpyrrole-4-yl group, 3-(2-phenylpropyl)pyrrole-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group, 1-dibenzofuranyl group, 2-dibenzofuranyl group, 3-dibenzofuranyl group, 4-dibenzofuranyl group, 1-dibenzothiophenyl group, 2-dibenzothiophenyl group, 3-dibenzothiophenyl group, 4-dibenzothiophenyl group, 1-silafluorenyl group, 2-silafluorenyl group, 3-silafluorenyl group, 4-silafluorenyl group, 1-germafluorenyl group, 2-germafluorenyl group, 3-germafluorenyl group and 4-germafluorenyl group.

Examples of the linear, branched or cyclic alkyl group having 1 to 30 carbon atoms in the formula (1) are a methyl group, ethyl group, propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neo-pentyl group, 1-methylpentyl group, 2-methylpentyl group, 1-pentylhexyl group, 1-butylpentyl group, 1-heptyloctyl group, 3-methylpentyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 1,2-dinitroethyl group, 2,3-dinitro-t-butyl group and 1,2,3-trinitropropyl group.

Examples of the alkoxy group having 1 to 30 carbon atoms in the formula (1) are a methoxy group, ethoxy group, propoxy group, butoxy group, pentyloxy group and hexyloxy group.

The aryloxy group having 6 to 30 ring carbon atoms in the formula (1) is represented by -OAr. Herein, Ar is specifically exemplified by the same as the above examples of the aromatic hydrocarbon group.

Examples of the aralkyl group having 7 to 30 carbon atoms in the formula (1) are a benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrorylmethyl group, 2-(1-pyroryl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group, and 1-chloro-2-phenylisopropyl group.

The haloalkyl group having 1 to 30 carbon atoms in the formula (1) is exemplified by a haloalkyl group provided by substituting the alkyl group having 1 to 30 carbon atoms with one or more halogen groups.

The haloalkoxy group having 1 to 30 carbon atoms in the formula (1) is exemplified by a haloalkoxy group provided by substituting the haloalkoxy group having 1 to 30 carbon atoms with one or more halogen groups.

The trialkylsilyl group having 3 to 30 carbon atoms in the formula (1) is exemplified by a trialkylsilyl group having the above examples of the alkyl group having 1 to 30 carbon atoms. Specific examples of the trialkylsilyl group are a trimethylsilyl group, triethylsilyl group, tri-n-butylsilyl group, tri-n-octylsilyl group, triisobutylsilyl group, dimethylethylsilyl group, dimethylisopropylsilyl group, dimethyl-n-propylsilyl group, dimethyl-n-butylsilyl group, dimethyl-t-butylsilyl group, and diethylisopropylsilyl group. Three alkyl groups in the trialkylsilyl group may be the same or different.

The dialkylarylsilyl group having 8 to 40 carbon atoms in the formula (1) is exemplified by a dialkylarylsilyl group having two of the examples of the alkyl group having 1 to 30 carbon atoms and one of the aromatic hydrocarbon group having 6 to 30 ring carbon atoms.

The alkyldiarylsilyl group having 13 to 50 carbon atoms in the formula (1) is exemplified by an alkyldiarylsilyl group having one of the examples of the alkyl group having 1 to 30 carbon atoms and two of the aromatic hydrocarbon group having 6 to 30 ring carbon atoms.

The triarylsilyl group having 18 to 60 carbon atoms in the formula (1) is exemplified by a triarylsilyl group having three of the aromatic hydrocarbon group having 6 to 30 ring carbon atoms.

The biscarbazole derivative represented by the formula (1) is preferably represented by the formula (2), (3) or (4).

A biscarbazole derivative represented by the formula (2) has a structure in which one carbazolyl group is bonded at its position 4 to a position 1 of the other carbazolyl group.

A biscarbazole derivative represented by the formula (3) has a structure in which one carbazolyl group is bonded at its position 4 to a position 3 of the other carbazolyl group.

A biscarbazole derivative represented by the formula (4) has a structure in which one carbazolyl group is bonded at its position 4 to a position 2 of the other carbazolyl group.

Among the biscarbazole derivatives represented by the formulae (2), (3) and (4), the biscarbazole derivative represented by the formula (3) or (4) is preferable.

L₃ represents a single bond or a divalent linking group. As the divalent linking group, L₃ is preferably a divalent group derived from a substituted or unsubstituted aromatic hydrocarbon compound having 6 to 30 ring carbon atoms, or a divalent group derived from a substituted or unsubstituted aromatic heterocyclic compound having 1 to 30 ring carbon atoms.

L₃ in the formula (1) is more preferably a single bond.

The aromatic heterocyclic group as A₁ or A₂ in the formula (1) is preferably a nitrogen-containing aromatic heterocyclic group. The nitrogen-containing aromatic heterocyclic group is preferably a heterocyclic group having a pyrimidine skeleton or a heterocyclic group having a triazine skeleton.

The heterocyclic group having the pyrimidine skeleton is exemplified by a substituted or unsubstituted pyrimidinyl group. Examples of the pyrimidinyl group are a 2-pyrimidinyl group, 4-pyrimidinyl group, 5-pyrimidinyl group and 6-pyrimidinyl group.

The heterocyclic group having the triazine skeleton is exemplified by a substituted or unsubstituted triazinyl group. The triazinyl group is a group formed from a triazine ring and has three kinds of 1,2,3-triazine, 1,2,4-triazine and 1,3,5-triazine. Examples of the triazinyl group are a 1,2,3-triazine-4-yl group, 1,2,4-triazine-3-yl group and 1,3,5-triazine-2-yl group.

It is speculated that, as compared with bonding of other nitrogen-containing aromatic heterocyclic groups such as an imidazopyridinyl group, bonding of the pyrimidinyl group or triazinyl group at A₁ or A₂ improves resistance of the biscarbazole derivative against holes and electrons.

L₁ and L₂ each independently represent a single bond or a divalent linking group.

At least one of L₁ and L₂ in the formula (1) is preferably a single bond, a divalent group derived from a substituted or unsubstituted aromatic hydrocarbon compound having 6 to 30 ring carbon atoms, or a divalent group derived from a substituted or unsubstituted aromatic heterocyclic compound having 1 to 30 ring carbon atoms.

When at least one of L₁ and L₂ is a single bond, hole transporting capability is improved.

When at least one of L₁ and L₂ is a divalent group derived from a substituted or unsubstituted aromatic hydrocarbon compound having 6 to 30 ring carbon atoms, or a divalent group derived from a substituted or unsubstituted aromatic heterocyclic compound having 1 to 30 ring carbon atoms, electron transporting capability tends to be improved.

Accordingly, it is desirable to appropriately select L₁ and L₂ in order to adjust balance of carrier transporting capability of the biscarbazole derivative. Thus, it is also effective to appropriately select L₁ and L₂ when the biscarbazole derivative according to the exemplary embodiment is used as a host material.

In the exemplary embodiment of the invention, examples of the substituent meant by "substituted or unsubstituted" are the above-described aromatic hydrocarbon group, aromatic heterocyclic group, alkyl group (linear or branched alkyl group, cycloalkyl group and haloalkyl group), alkoxy group, aryloxy group, aralkyl group, haloalkoxy group, alkylsilyl group, dialkylarylsilyl group, alkyldiarylsilyl group, triarylsilyl group, alkenyl group, alkynyl group, halogen atom, cyano group, hydroxyl group, nitro group and carboxy group.

In the exemplary embodiment of the invention, "unsubstituted" in a "substituted or unsubstituted XX group" means that a hydrogen atom of the XX group is not substituted by the above-described substituents.

Herein, "a to b carbon atoms" in the description of "substituted or unsubstituted XX group having a to b carbon atoms" represent carbon atoms of an unsubstituted XX group and does not include carbon atoms of a substituted XX group.

In a later-described compound or a partial structure thereof, the same applies to the description of "substituted or unsubstituted."

In the exemplary embodiment of the invention, "carbon atoms forming a ring (ring carbon atoms)" mean carbon atoms forming a saturated ring, unsaturated ring, or aromatic ring. "Atoms forming a ring (ring atoms)" mean carbon atoms and hetero atoms forming a hetero ring including a saturated ring, unsaturated ring, or aromatic ring.

In the exemplary embodiment of the invention, a "hydrogen atom" means isotopes having different neutron numbers and specifically encompasses protium, deuterium and tritium.

Examples of the biscarbazole derivative according to the exemplary embodiment are as follows. However, the invention is not limited to the biscarbazole derivative having these structures.

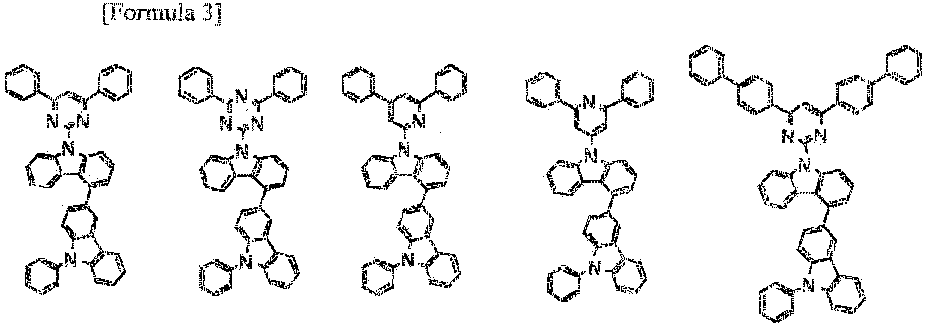

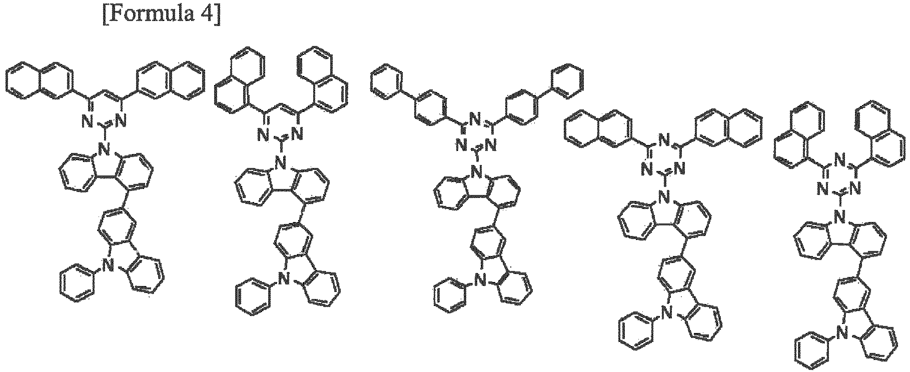

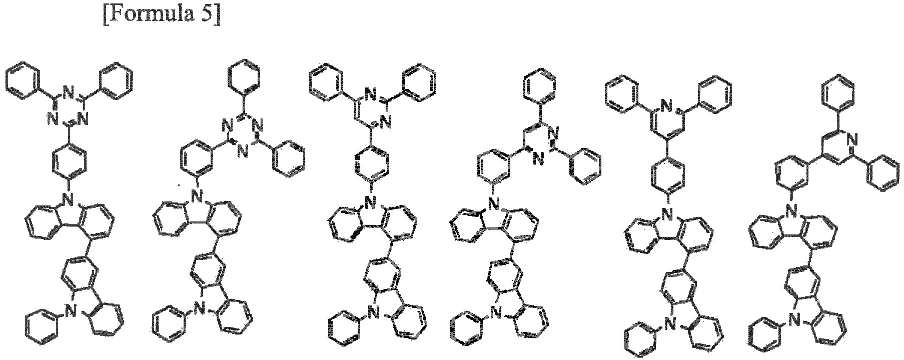

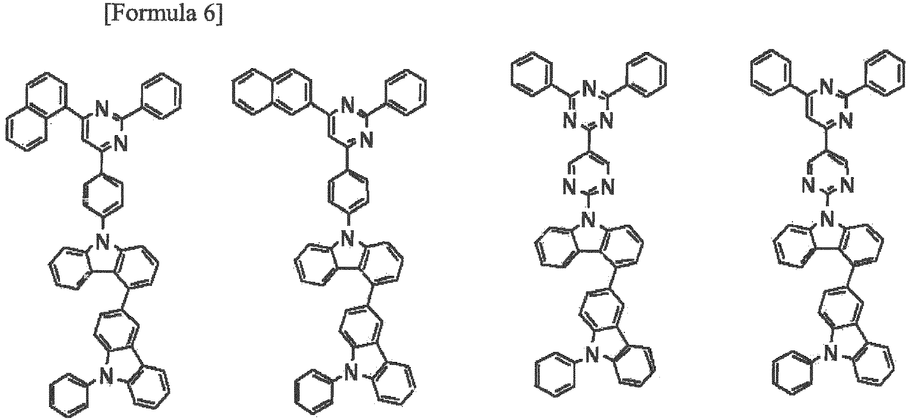

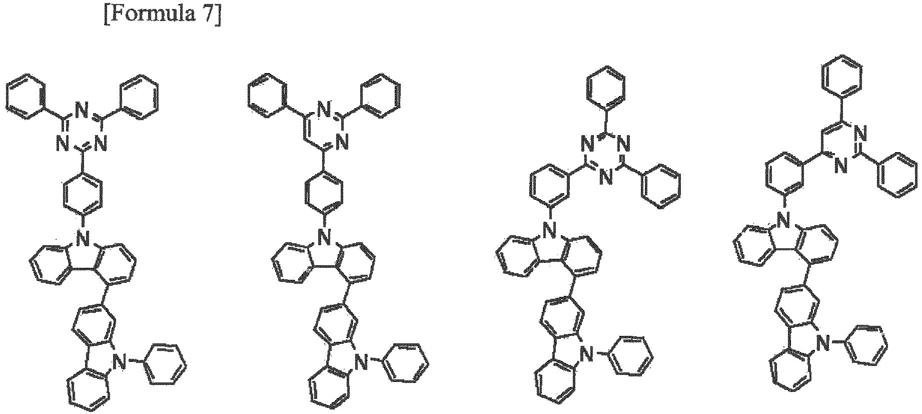

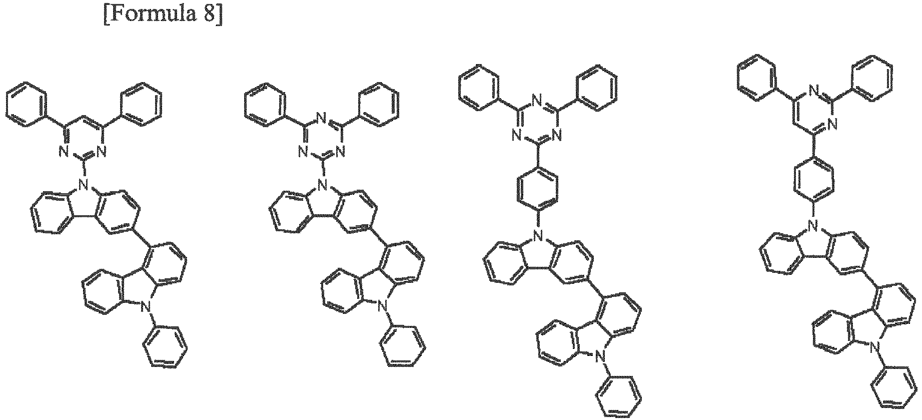

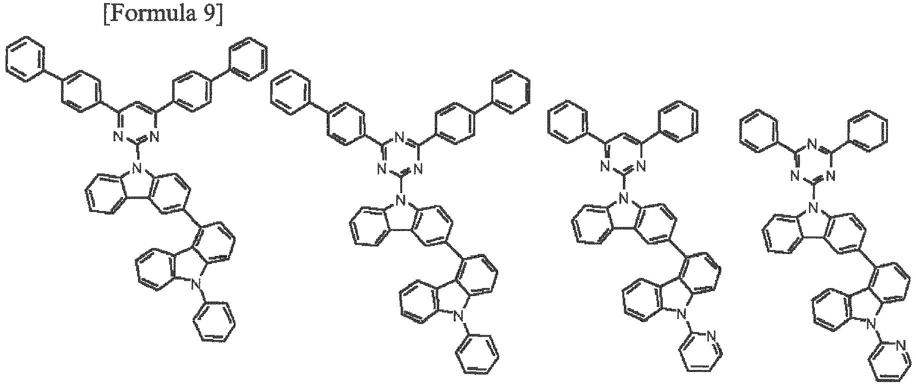

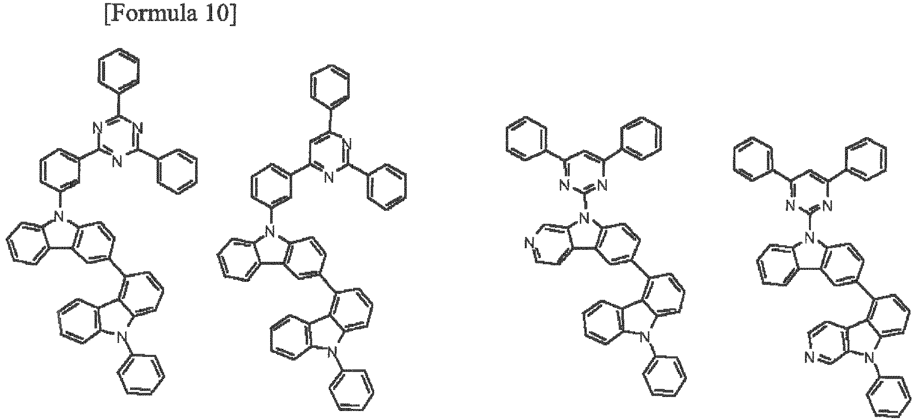

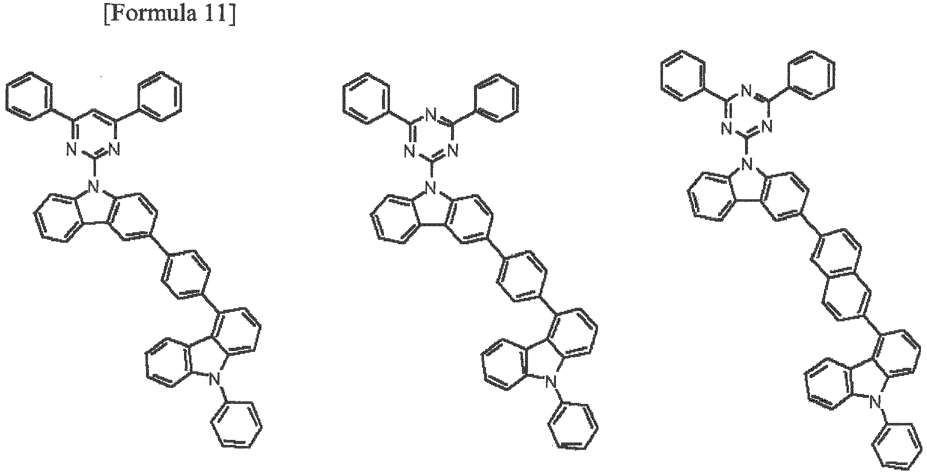

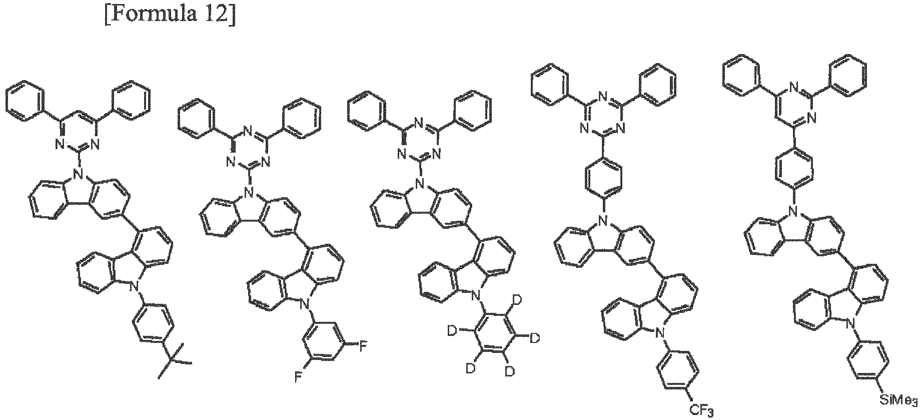

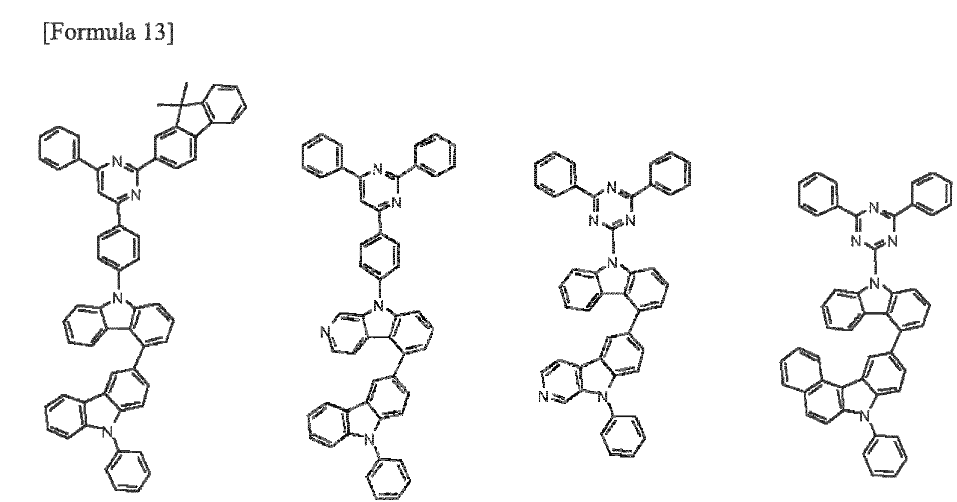

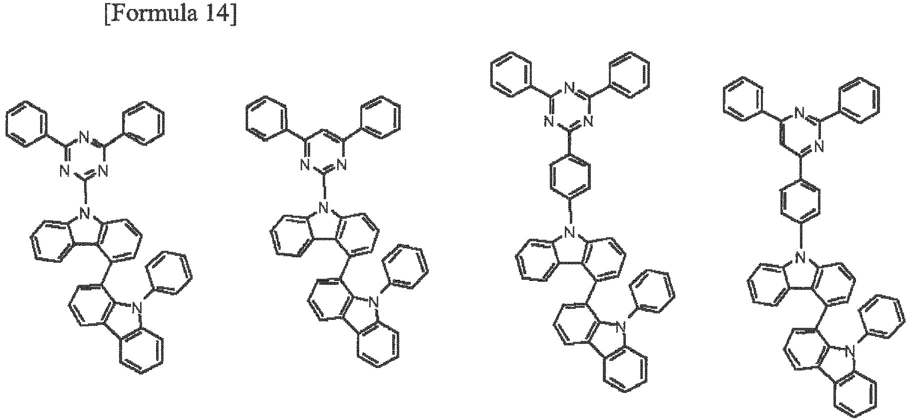

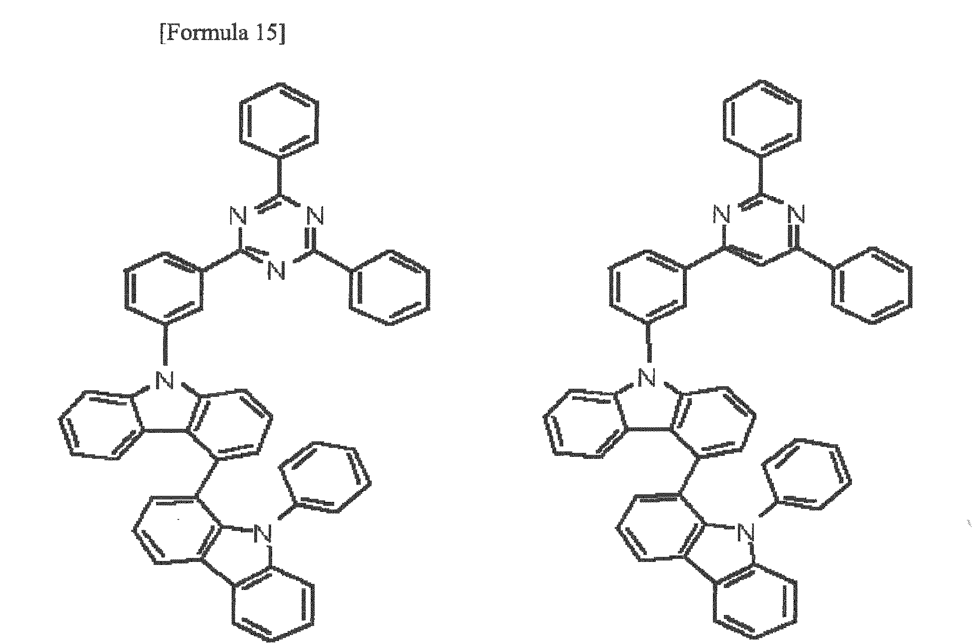

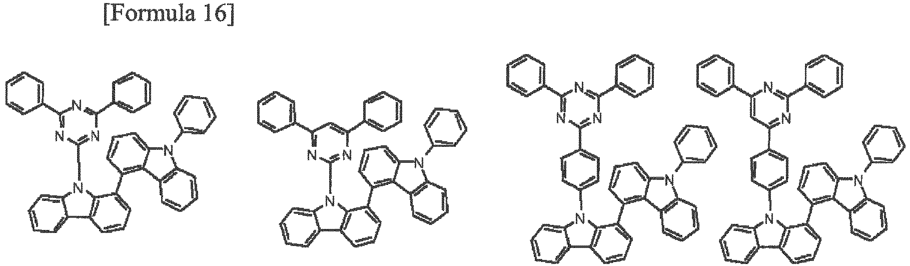

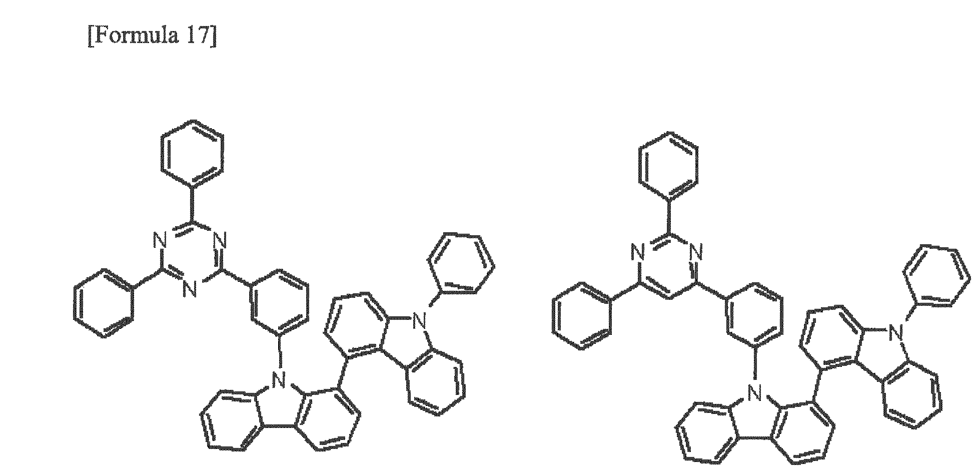

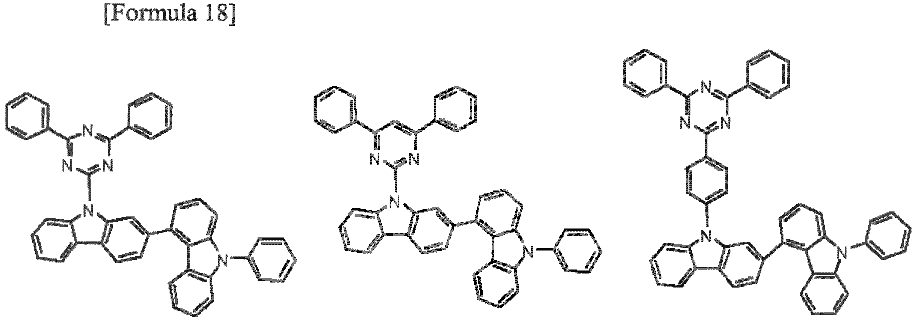

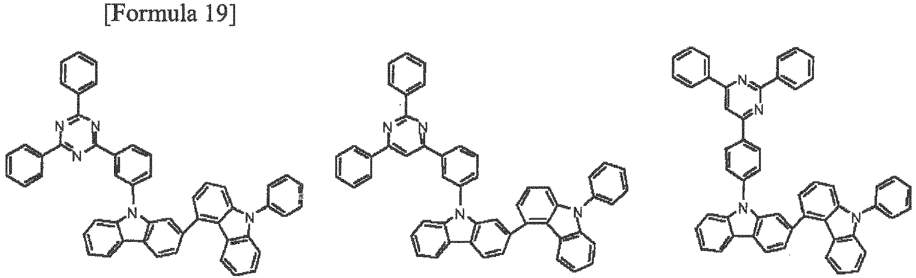

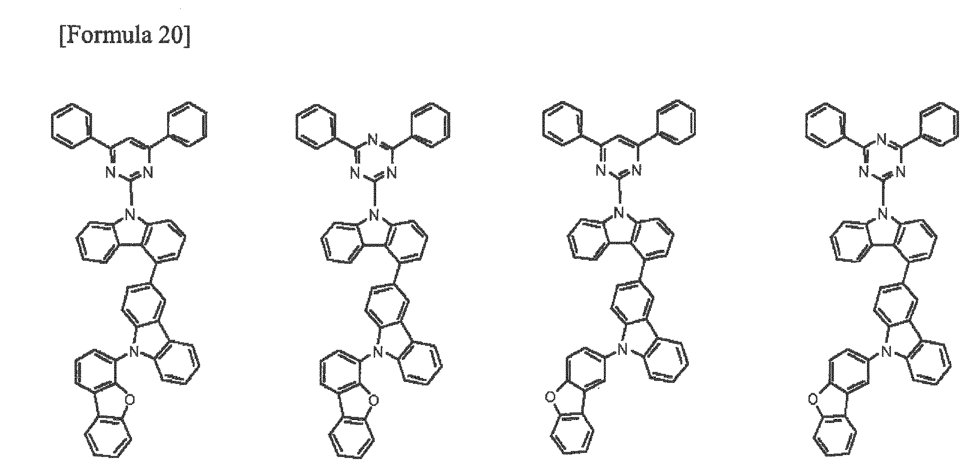

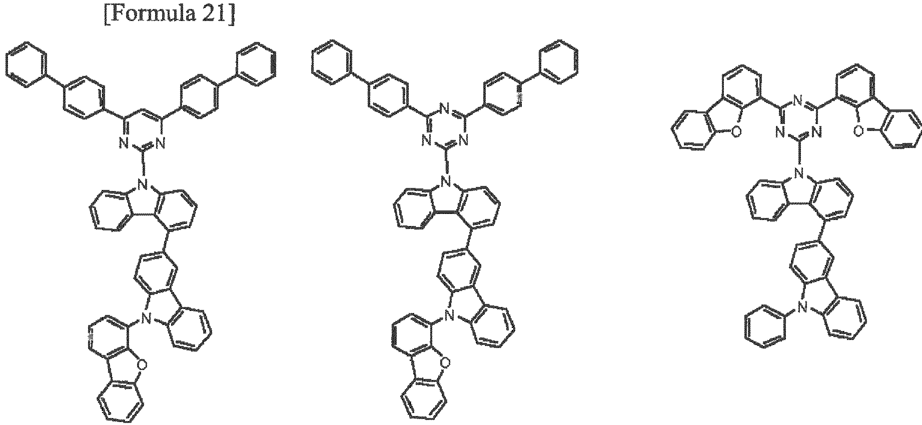

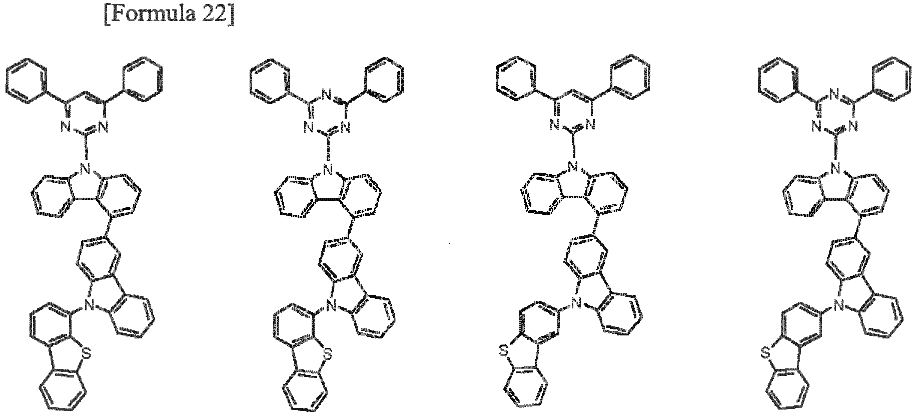

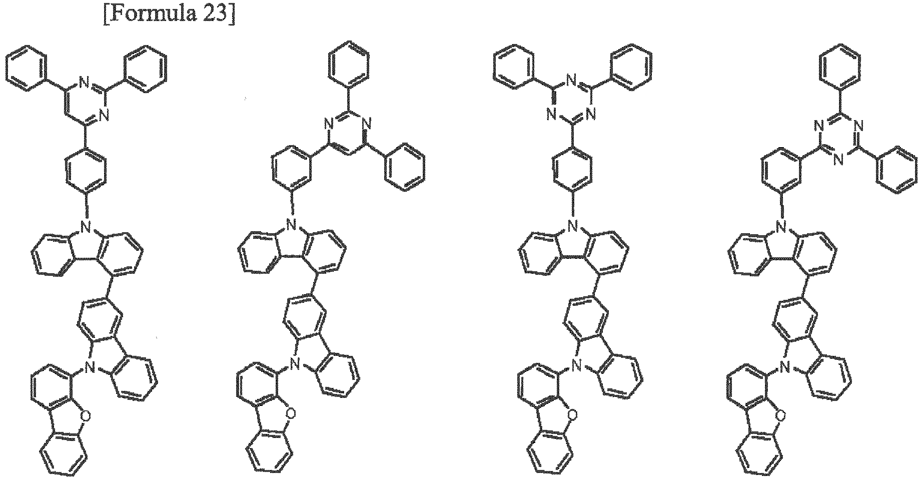

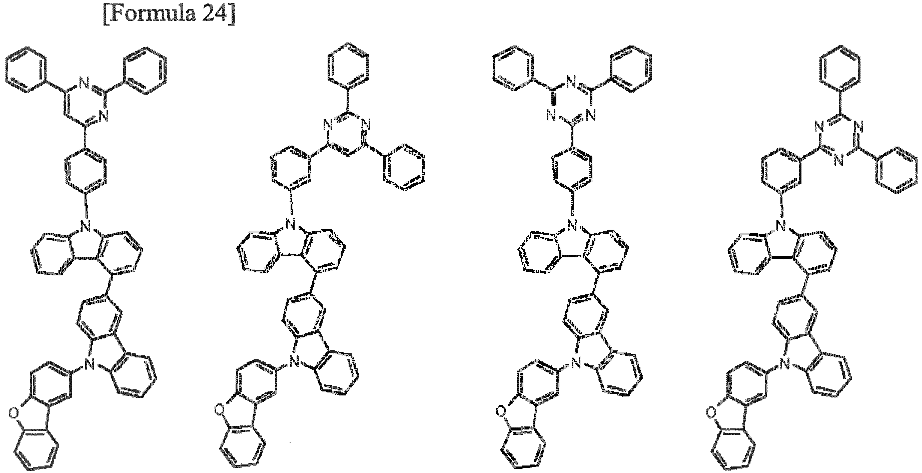

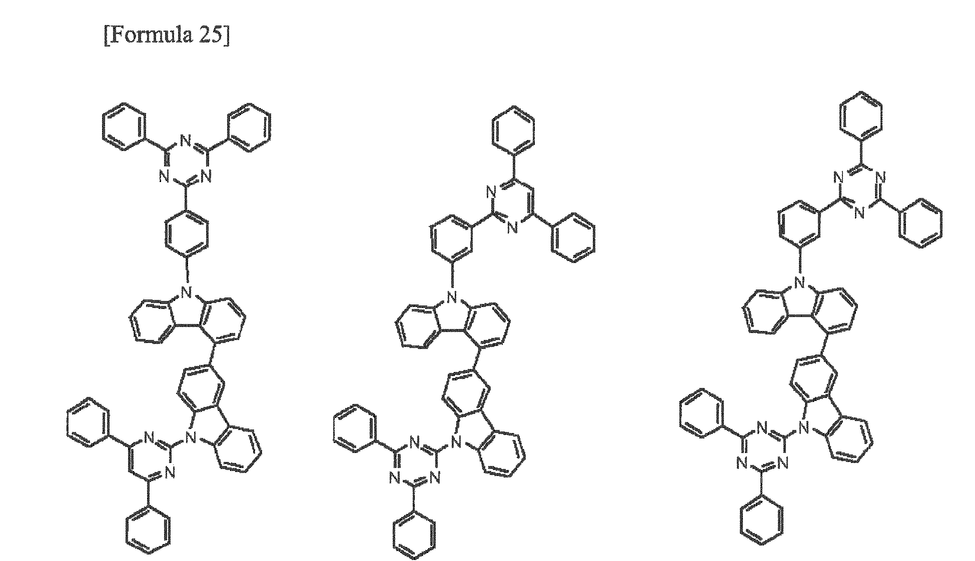

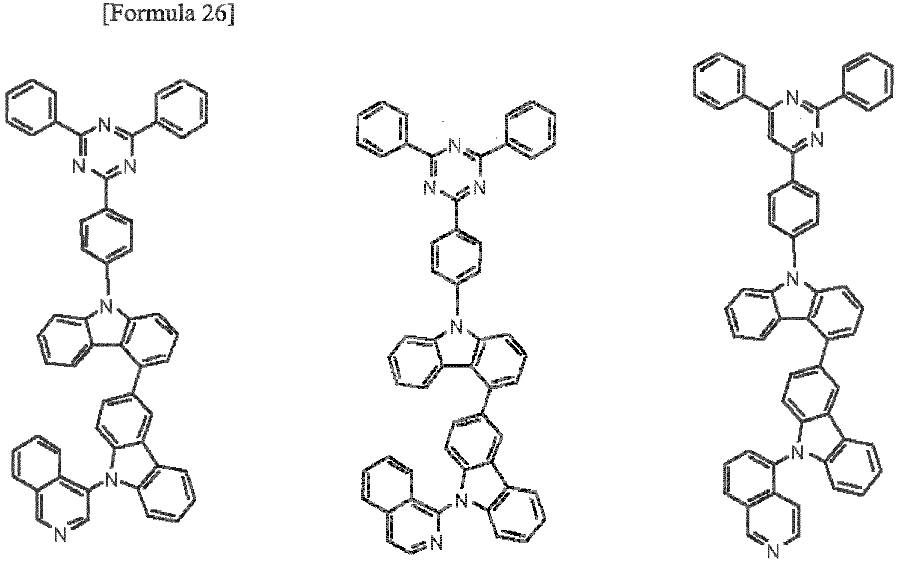

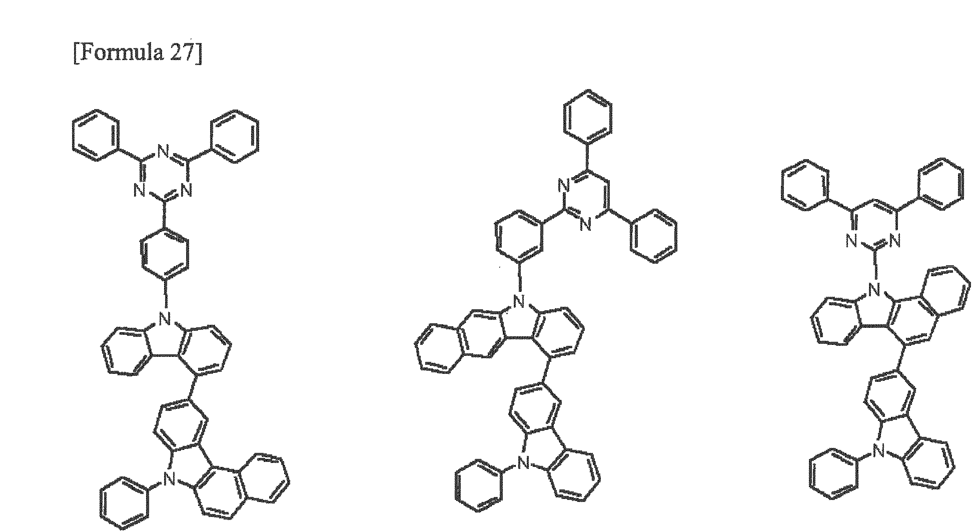

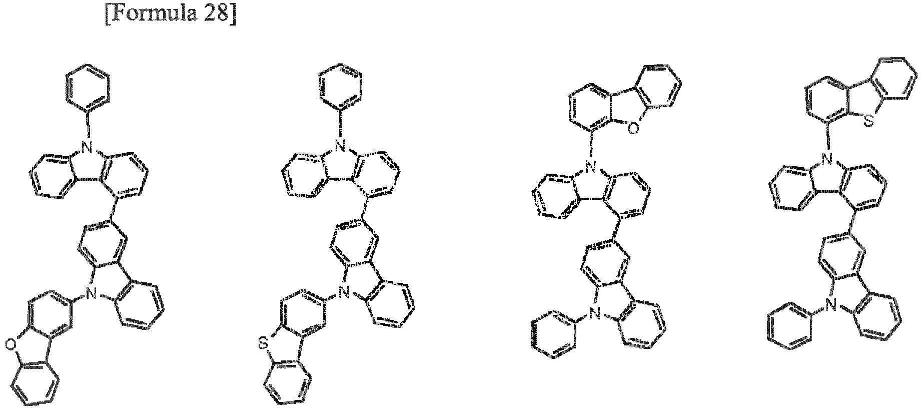

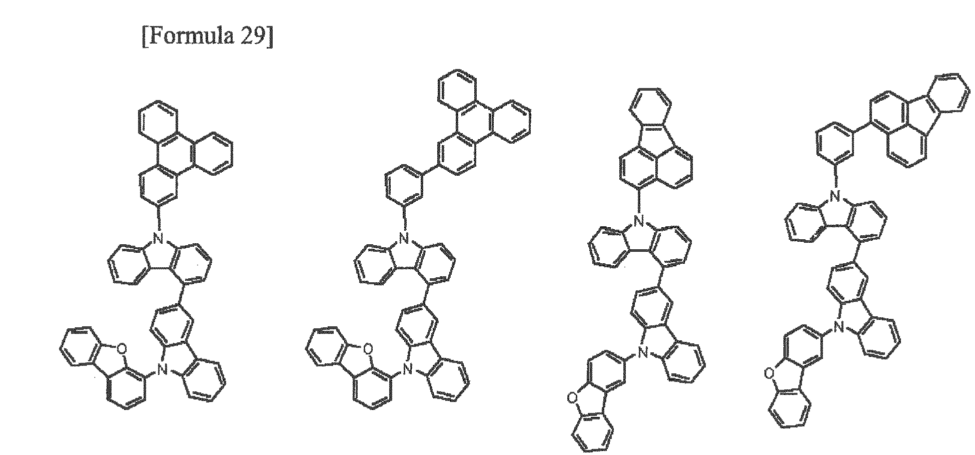

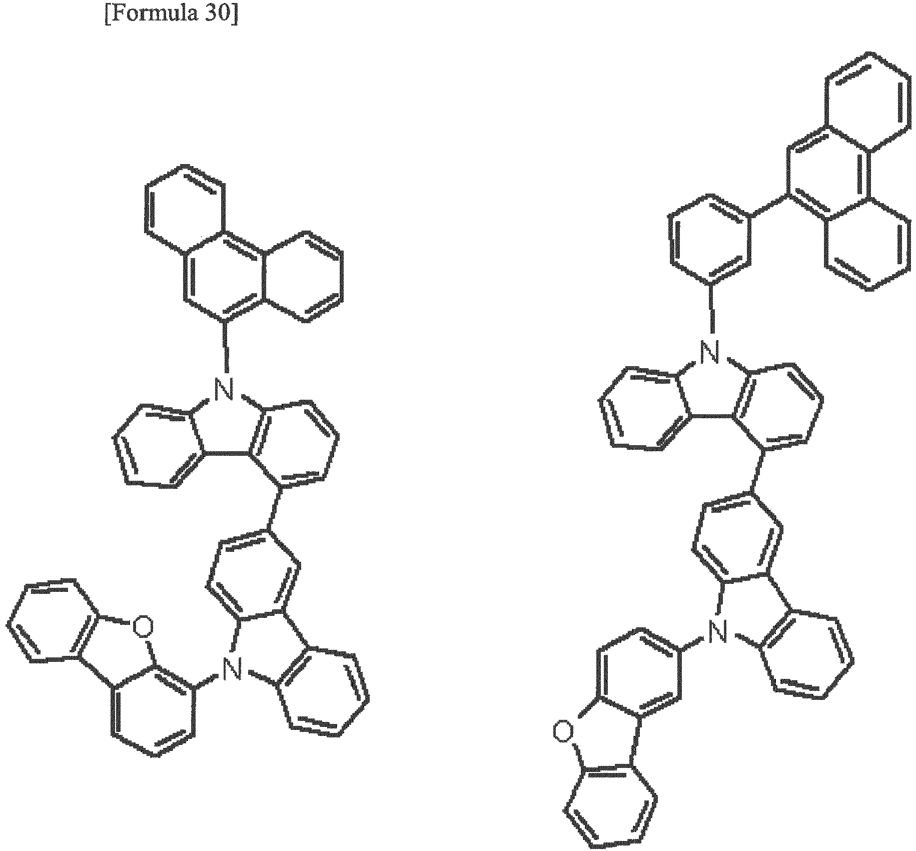

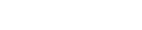

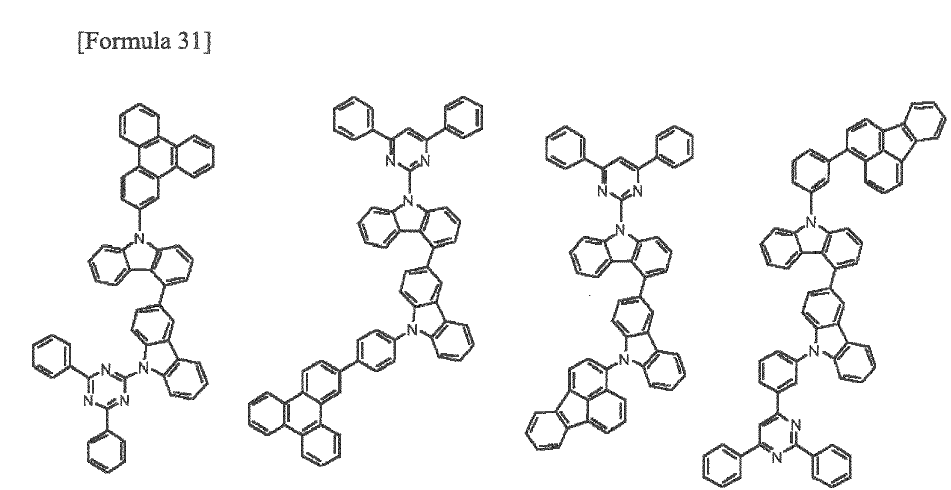

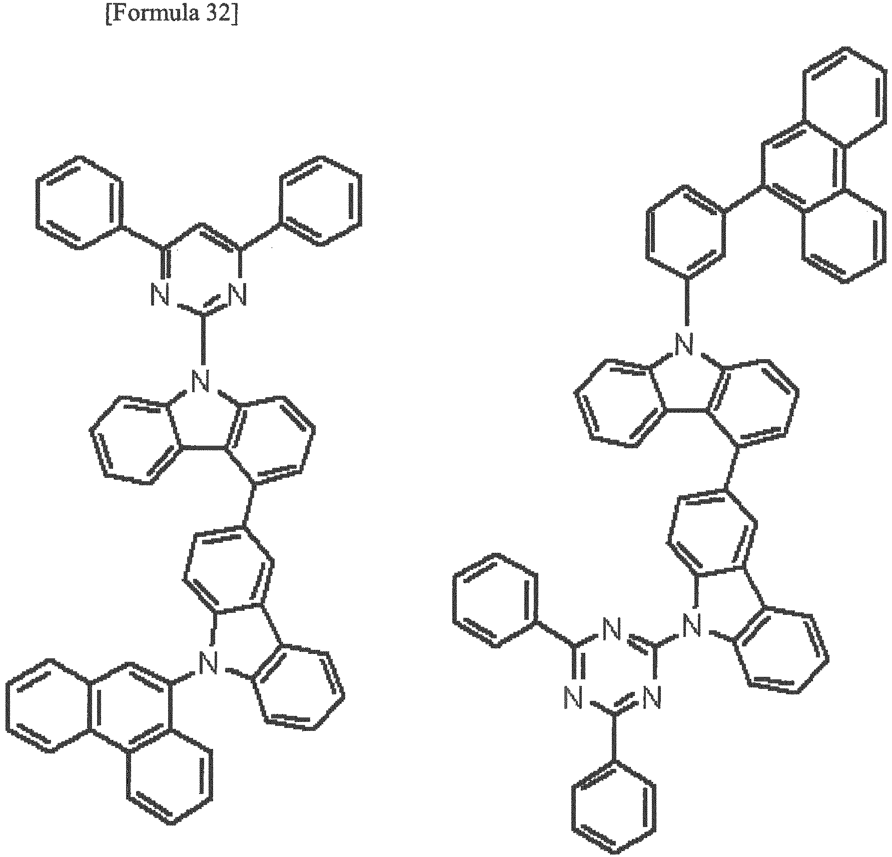

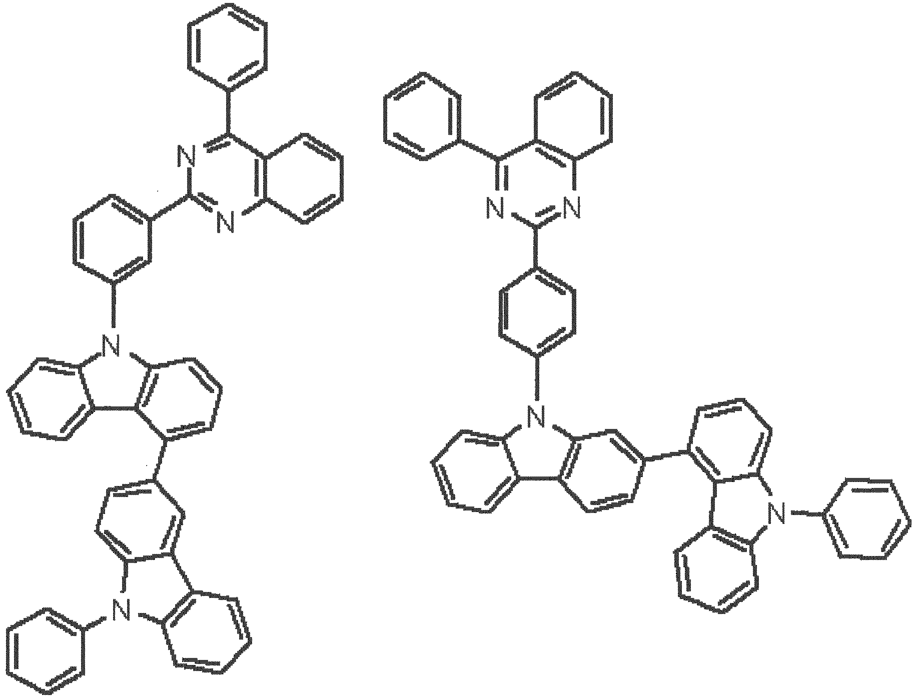

### Organic-EL-Device Material

The biscarbazole derivative according to the exemplary embodiment is usable as an organic-EL-device material. The organic-EL-device material according to the exemplary embodiment may only contain the biscarbazole derivative according to the above exemplary embodiment, or alternatively, may contain another compound in addition to the biscarbazole derivative according to the above exemplary embodiment.

### Arrangement of Organic EL Device

The organic EL device according to this exemplary embodiment includes an organic compound layer between a cathode and an anode.

The biscarbazole derivative according to the above exemplary embodiment is contained in the organic compound layer. The organic compound layer is formed using the organic-EL-device material containing the biscarbazole derivative according to the exemplary embodiment.

The organic compound layer has at least one layer of an organic thin-film layer formed of an organic compound. The organic thin-film layer may contain an inorganic compound.

In the organic EL device according to the exemplary embodiment, at least one layer of the organic thin-film layer(s) is the emitting layer. Accordingly, the organic compound layer may be provided by a single emitting layer. Alternatively, the organic thin-film layer may be provided by layers applied in a known organic EL device such as a hole injecting layer, a hole transporting layer, an electron injecting layer, an electron transporting layer, a hole blocking layer, an electron blocking layer. When the organic thin-film layer is provided by plural layers, the biscarbazole derivative according to the above exemplary embodiment is contained in at least one of the layers.

The followings are representative arrangement examples of an organic EL device:
(a) anode / emitting layer / cathode;
(b) anode / hole injecting·transporting layer / emitting layer / cathode;
(c) anode / emitting layer / electron injecting·transporting layer / cathode;
(d) anode / hole injecting·transporting layer / emitting layer / electron injecting·transporting layer / cathode; and
(e) anode / hole injecting·transporting layer / emitting layer / blocking layer / electron injecting·transporting layer / cathode.

While the arrangement (d) is preferably used among the above arrangements, the arrangement of the invention is not limited to the above arrangements.

It should be noted that the aforementioned "emitting layer" is an organic layer having an emission function, the organic layer including a host material and a dopant material when employing a doping system. Herein, the host material mainly has a function to promote recombination of electrons and holes and to confine excitons in the emitting layer while the dopant material has a function to efficiently emit the excitons obtained by the recombination. In a phosphorescent organic EL device, the host material mainly has a function to confine excitons generated in the dopant within the emitting layer.

The "hole injecting/transporting layer" means "at least one of a hole injecting layer and a hole transporting layer" while the "electron injecting/transporting layer" means "at least one of an electron injecting layer and an electron transporting layer." Herein, when the hole injecting layer and the hole transporting layer are provided, the hole injecting layer is preferably close to the anode. When the electron injecting layer and the electron transporting layer are provided, the electron injecting layer is preferably close to the cathode.

In the exemplary embodiment of the invention, the electron transporting layer means an organic layer having the highest electron mobility among organic layer(s) providing an electron transporting zone existing between the emitting layer and the cathode. When the electron transporting zone is provided by a single layer, the single layer is the electron transporting layer. Moreover, in the phosphorescent organic EL device, a blocking layer having an electron mobility that is not always high may be provided as shown in the arrangement (e) between the emitting layer and the electron transporting layer in order to prevent diffusion of exciton energy generated in the emitting layer. Thus, the organic layer adjacent to the emitting layer does not always correspond to the electron transporting layer.

Fig. 1 schematically shows an exemplary arrangement of the organic EL device according to the exemplary embodiment of the invention.

An organic EL device 1 includes a transparent substrate 2, an anode 3, a cathode 4 and an organic compound layer 10 interposed between the anode 3 and the cathode 4.

The organic thin-film layer 10 sequentially includes a hole injecting layer 5, a hole transporting layer 6, an emitting layer 7, an electron transporting layer 8 and an electron injection layer 9 on the anode 3.

### Transparent Substrate

The organic EL device according to this exemplary embodiment is prepared on a light-transmissive substrate. The light-transmissive plate, which supports the organic EL device, is preferably a smoothly-shaped substrate that transmits 50% or more of light in a visible region of 400 nm to 700 nm.

Specific examples of the substrate are a glass plate and a polymer plate.

For the glass plate, materials such as soda-lime glass, barium/strontium-containing glass, lead glass, aluminosilicate glass, borosilicate glass, barium borosilicate glass and quartz can be used.

For the polymer plate, materials such as polycarbonate, acryl, polyethylene terephthalate, polyether sulfide and polysulfone can be used.

### Anode and Cathode

The anode of the organic EL device is used for injecting holes into the hole injecting layer, the hole transporting layer or the emitting layer. It is effective that the anode has a work function of 4.5 eV or more.

Exemplary materials for the anode are alloys of indium-tin oxide (ITO), tin oxide (NESA), indium zinc oxide, gold, silver, platinum and copper.

An anode can be prepared by forming a thin film out of these electrode materials by vapor deposition, sputtering, or the like.

When light from the emitting layer is to be emitted through the anode as in this embodiment, the anode preferably transmits more than 10% of the light in the visible region. Sheet resistance of the anode is preferably several hundreds Ω/ square or lower. Although depending on the material of the anode, thickness of the anode is typically in a range of 10 nm to 1 µm, and preferably in a range of 10 nm to 200 nm.

The cathode is preferably formed of a material with smaller work function in order to inject electrons into the electron injecting layer, the electron transporting layer and the emitting layer.

Although a material for the cathode is subject to no specific limitation, examples of the material are indium, aluminum, magnesium, alloy of magnesium and indium, alloy of magnesium and aluminum, alloy of aluminum and lithium, alloy of aluminum, scandium and lithium, alloy of magnesium and silver and the like.

Like the anode, the cathode may be made by forming a thin film from the above materials through a method such as vapor deposition or sputtering. In addition, the light may be emitted through the cathode. When light from the emitting layer is to be emitted through the cathode, the cathode preferably transmits more than 10% of the light in the visible region.

Sheet resistance of the cathode is preferably several hundreds Ω per square or lower.

Although depending on the material of the anode, thickness of the anode is typically in a range of 10 nm to 1 µm, and preferably in a range of 50 nm to 200 nm.

### Emitting Layer

The emitting layer of the organic EL device has a function for providing conditions for recombination of the electrons and the holes to emit light.

The emitting layer is preferably a molecular deposit film.

The molecular deposit film means a thin film formed by depositing a material compound in gas phase or a film formed by solidifying a material compound in a solution state or in liquid phase. The molecular deposit film is typically distinguished from a thin film formed by the LB method (molecular accumulation film) by differences in aggregation structures, higher order structures and functional differences arising therefrom.

As disclosed in JP-A-57-51781, the emitting layer can be formed from a thin film formed by spin coating or the like, the thin film being formed from a solution prepared by dissolving a binder (e.g. a resin) and a material compound in a solvent.

### Host Material

The host material is preferably the biscarbazole derivative according to the exemplary embodiment. As described above, the biscarbazole derivative according to the exemplary embodiment exhibits an excellent resistance to holes and electrons. Accordingly, use of the biscarbazole derivative as the host material can improve durability of the organic EL device.

### Dopant Material

The dopant material is selected from a known fluorescent material that emits fluorescence or a known phosphorescent material that emits phosphorescence.

The fluorescent material used as the dopant material (hereinafter, referred to as a fluorescent dopant material) is selected from a fluoranthene derivative, pyrene derivative, arylacetylene derivative, fluorene derivative, boron complex, perylene derivative, oxadiazole derivative, and anthracene derivative. The fluoranthene derivative, pyrene derivative and boron complex are preferable.

The phosphorescent material is preferable as the dopant material of the organic EL device according to the exemplary embodiment. The phosphorescent material used as the dopant material (hereinafter, referred to as a phosphorescent dopant material) preferably contains a metal complex. The metal complex preferably contains: a metal atom selected from iridium (Ir), platinum (Pt), osmium (Os), gold (Au), rhenium (Re) and ruthenium (Ru); and a ligand. Particularly, an ortho-metalated complex in which the ligand and the metal atom form an ortho-metal bond is preferable. As the phosphorescent dopant material, an ortho-metalated complex containing a metal selected from the group consisting of iridium (Ir), osmium (Os) and platinum (Pt) is preferable since a phosphorescent quantum yield is high and an external quantum efficiency of an emitting device is improvable. In terms of the luminous efficiency, a metal complex including the ligand selected from phenyl quinoline, phenyl isoquinoline, phenyl pyridine, phenyl pyrimidine, phenyl pyrazine and phenyl imidazole is preferable.

Examples of the phosphorescent dopant material are shown below.

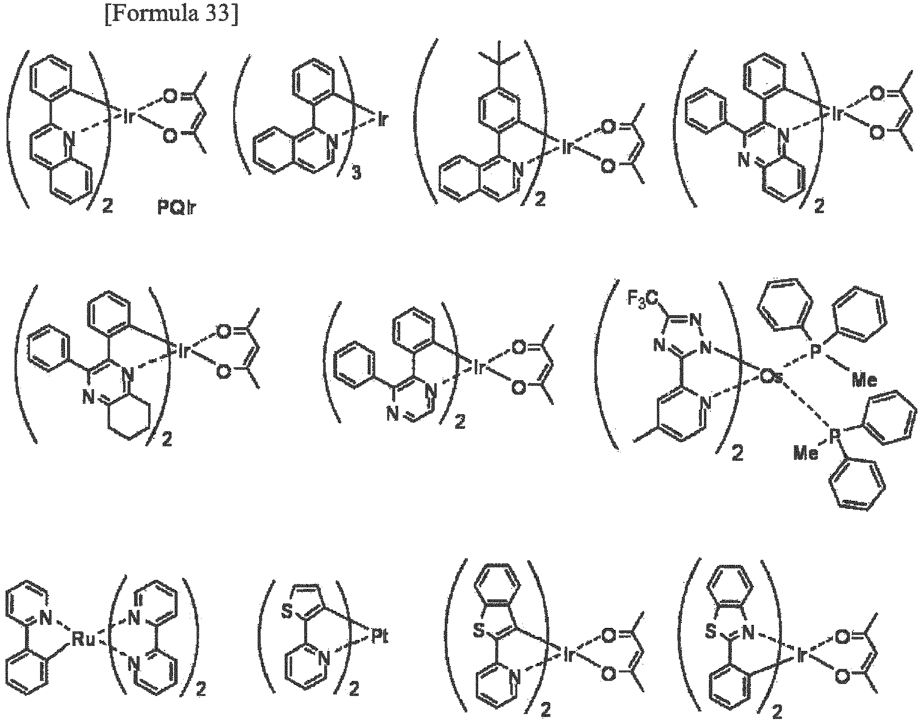

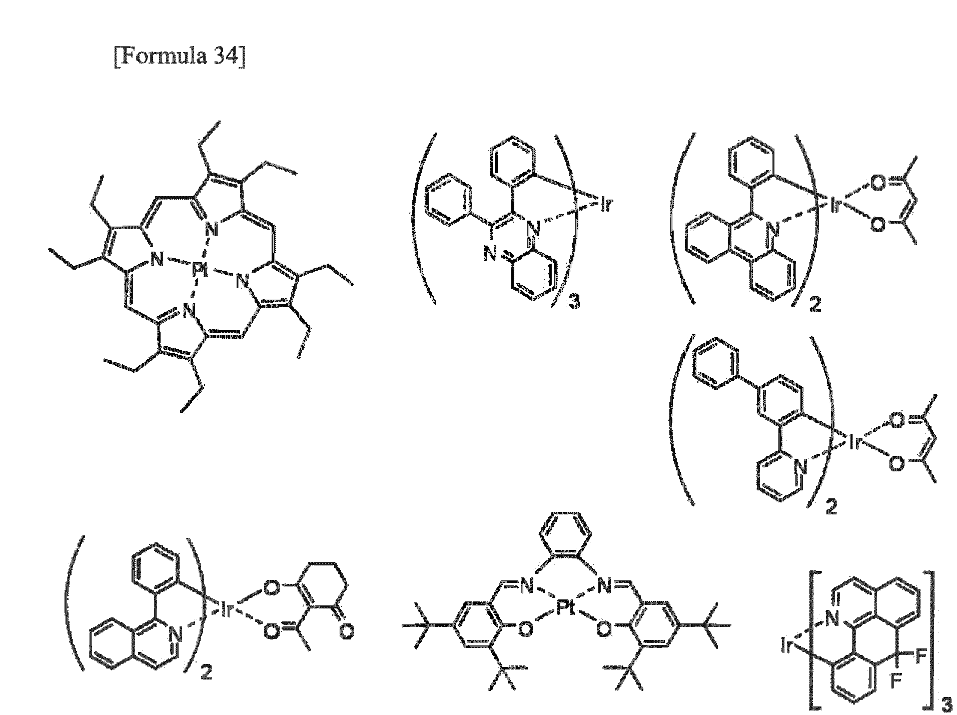

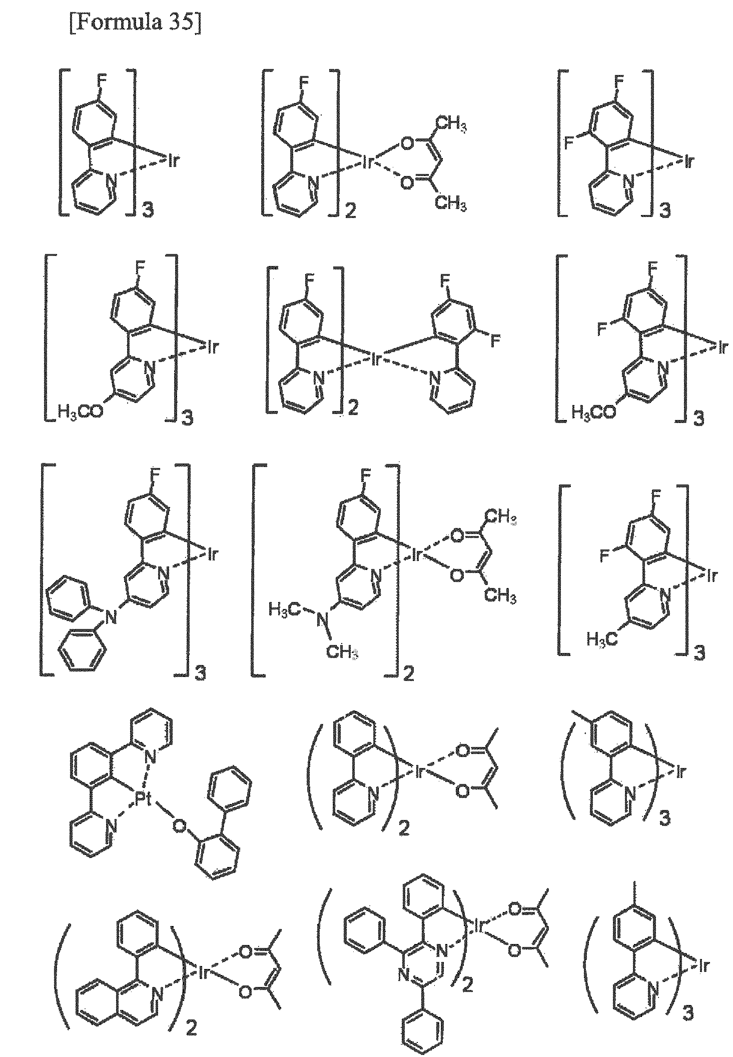

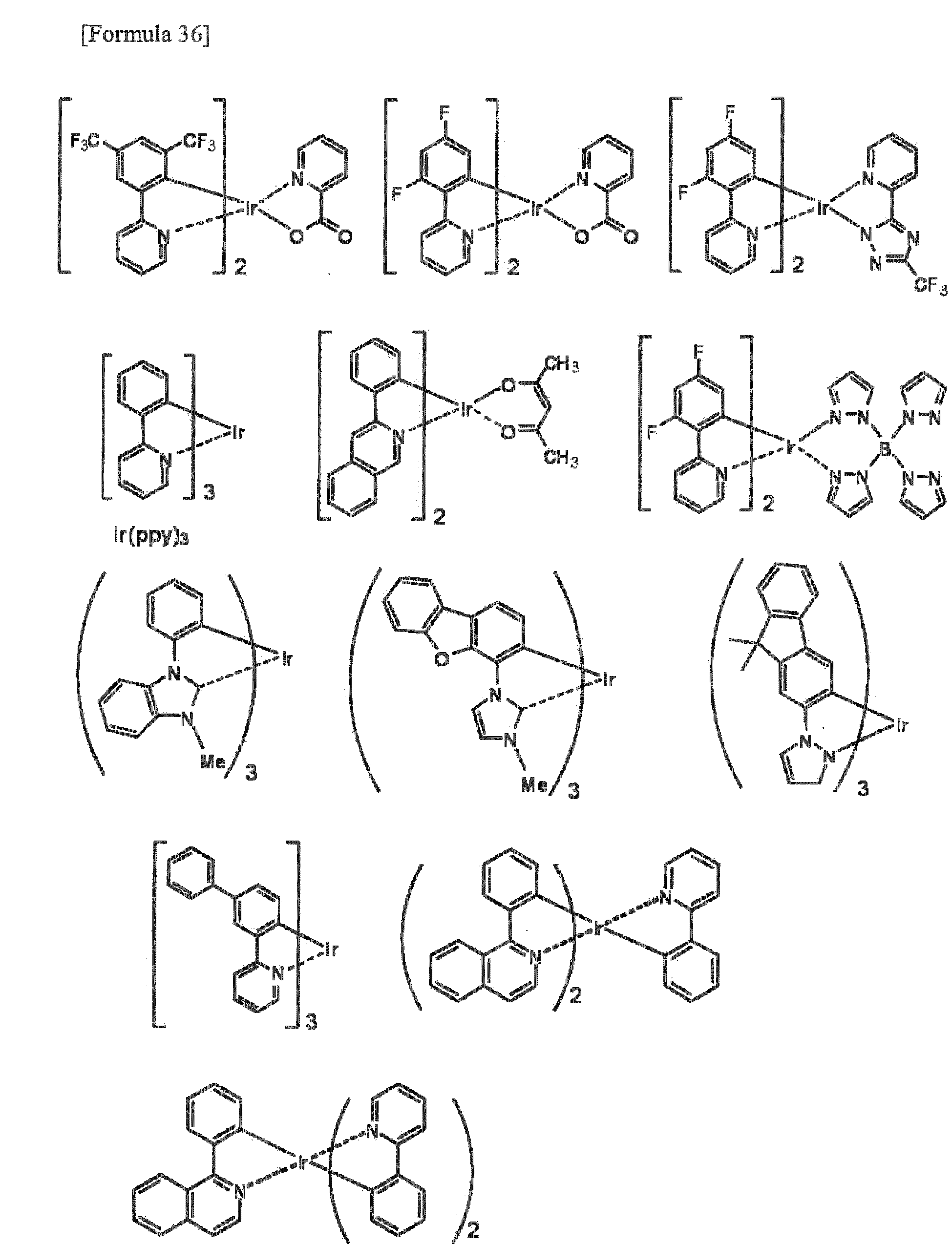

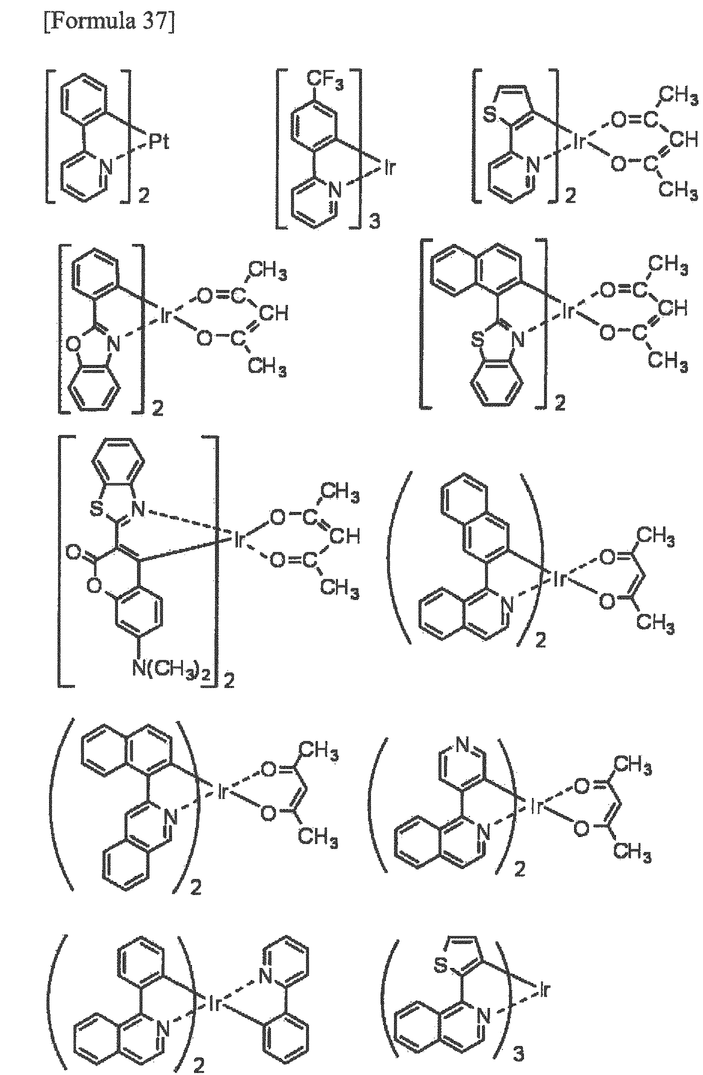

Having the aforementioned structure, the biscarbazole derivative according to the exemplary embodiment exhibits a larger triplet energy than the biscarbazole derivative in which the carbazolyl groups are bonded to each other at their positions 3. Accordingly, when the biscarbazole derivative according to the exemplary embodiment is used as the phosphorescent host material in the emitting layer of the organic EL device according to the exemplary embodiment of the invention, a phosphorescent material emitting phosphorescence in a wavelength region from green to blue is preferably used as the phosphorescent dopant material. When the phosphorescent dopant material and phosphorescent host material are thus combined in the phosphorescent organic EL device, it is presumed that transfer of triplet excitons from the phosphorescent dopant material to the phosphorescent host material is blocked, whereby triplet excitons of the phosphorescent dopant material are efficiently confined.

### Other Materials Contained in Emitting Layer

In the organic EL device according to this exemplary embodiment, the emitting layer preferably further contains an aromatic amine derivative. When the emitting layer contains the aromatic amine derivative in addition to the biscarbazole derivative according to the exemplary embodiment (host material) and the dopant material, hole injection and hole transport are assisted, so that holes and electrons can easily be balanced in the emitting layer.

Examples of the aromatic amine derivative are compounds used in the following hole injecting/transporting layer.

### Hole Injecting/Transporting Layer

The hole injecting/transporting layer helps injection of holes to the emitting layer and transports the holes to an emitting region. The hole injecting/transporting layer exhibits a large hole mobility and a small ionization energy.

The hole injecting/transporting layer may be provided by a hole injecting layer or a hole transporting layer, or alternatively, may be provided by a laminate of a hole injecting layer and a hole transporting layer.

A material for forming the hole injection/transport layer is preferably a material for transporting the holes to the emitting layer at a lower electric field intensity. For instance, an aromatic amine compound represented by the following formula (Al) is preferably used.

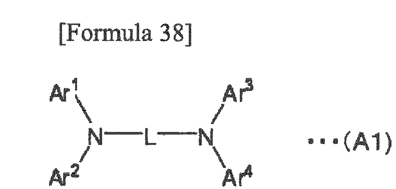

In the formula (Al), Ar¹ to Ar⁴ each represent: an aromatic hydrocarbon group having 6 to 50 ring carbon atoms, an aromatic heterocyclic group having 2 to 40 ring carbon atoms, a group provided by bonding the aromatic hydrocarbon group to the aromatic heterocyclic group, or a group provided by bonding the aromatic hydrocarbon group to the aromatic heterocyclic group.

Note that the aromatic hydrocarbon group and the aromatic heterocyclic group described herein may have a substituent.

In the formula (Al), L is a linking group and represents a divalent aromatic hydrocarbon group having 6 to 50 ring carbon atoms, a divalent aromatic heterocyclic group having 5 to 50 ring carbon atoms, and a divalent group obtained by bonding two or more of the aromatic hydrocarbon group or aromatic heterocyclic group to each other through a single bond, an ether bond, a thioether bond, an alkylene group having 1 to 20 carbon atoms, an alkenylene group having 2 to 20 carbon atoms, or an amino group. Note that the divalent aromatic hydrocarbon group and the divalent aromatic heterocyclic group described herein may have a substituent.

Examples of the compound represented by the formula (Al) are shown below. However, the compound represented by the formula (Al) is not limited thereto.

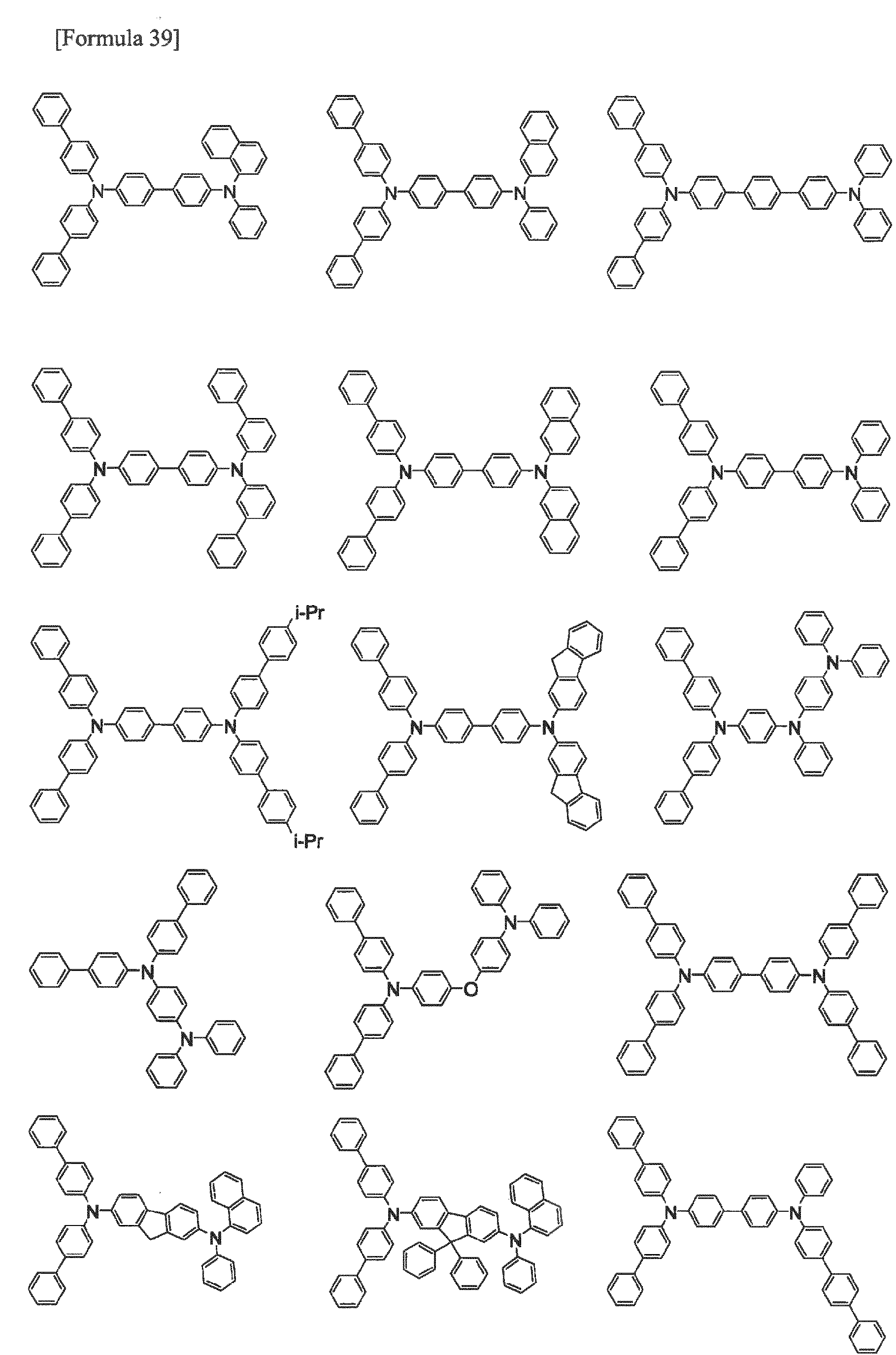

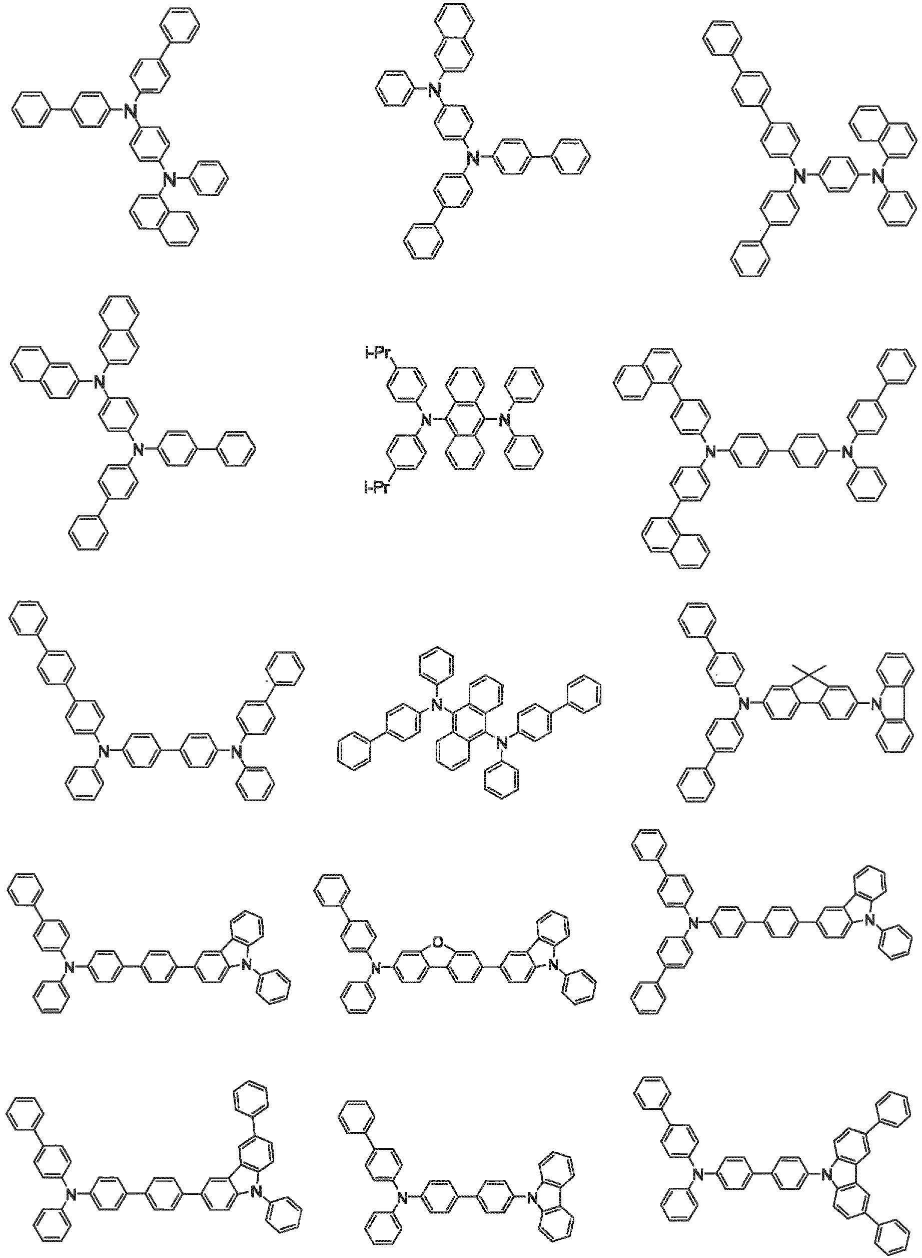

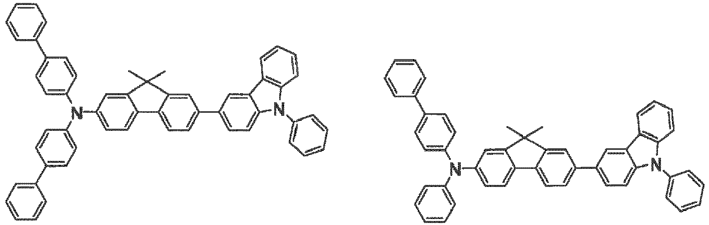

Aromatic amine represented by the following formula (A2) can also be preferably used for forming the hole injecting/transporting layer.

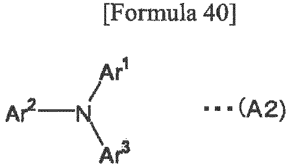

In the above formula (A2), Ar¹ to Ar³ each represent the same as Ar¹ to Ar⁴ of the above formula (Al). Examples of the compound represented by the formula (A2) are shown below. However, the compound represented by the formula (A2) is not limited thereto.

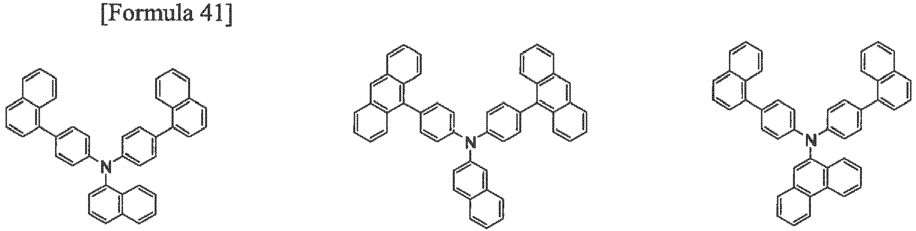

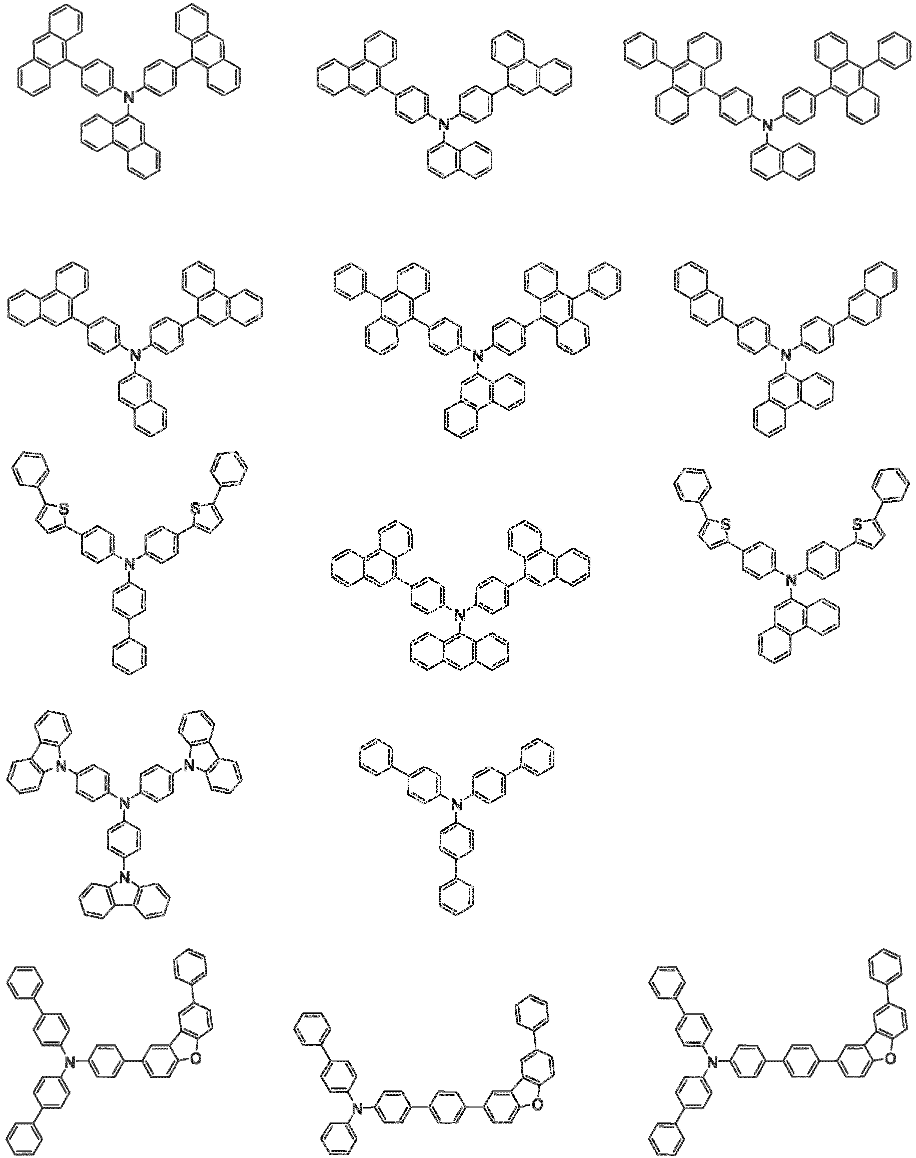

### Electron Injecting/Transporting Layer

The electron injection/transport layer helps injection of the electron to the luminescent layer and has a high electron mobility. The electron injecting layer is provided for adjusting energy level, by which, for instance, sudden changes of the energy level can be reduced. The electron injection/transport layer includes at least one of the electron injecting layer and the electron transporting layer.

The organic EL device according to the invention preferably includes the electron injecting layer between the emitting layer and the cathode, and the electron injecting layer preferably contains a nitrogen-containing cyclic derivative as a main component. The electron injecting layer may serve as the electron transporting layer.

It should be noted that "as a main component" means that the nitrogen-containing cyclic derivative is contained in the electron injecting layer at a content of 50 mass% or more.

A preferable example of an electron transporting material for forming the electron injecting layer is an aromatic heterocyclic compound having in the molecule at least one heteroatom. Particularly, a nitrogen-containing cyclic derivative is preferable. The nitrogen-containing cyclic derivative is preferably an aromatic cyclic compound having a nitrogen-containing six-membered or five-membered ring skeleton.

The nitrogen-containing cyclic derivative is preferably exemplified by a nitrogen-containing cyclic metal chelate complex represented by the following formula (B1).

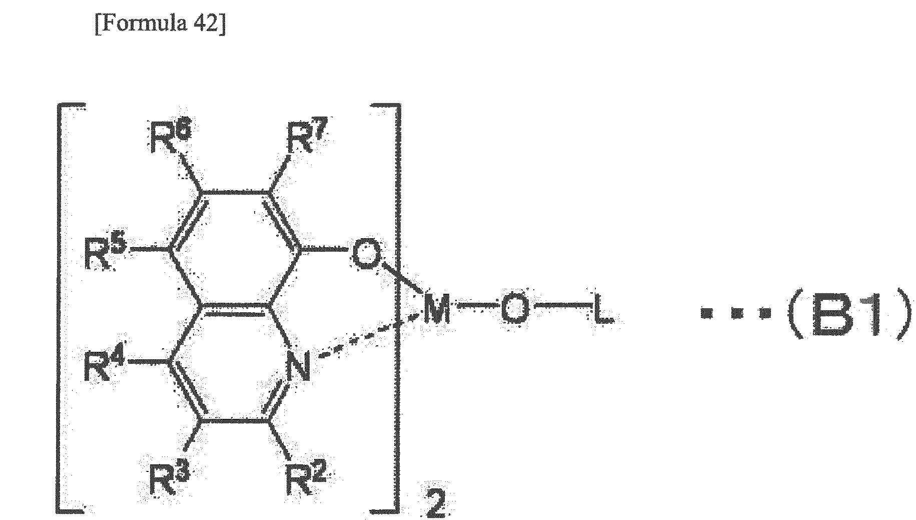

In the formula (B1), R² to R⁷ independently represent a hydrogen atom, a halogen atom, an oxy group, an amino group, a hydrocarbon group having 1 to 40 carbon atoms, an alkoxy group, an aryloxy group, an alkoxycarbonyl group, or an aromatic heterocyclic group, which may have a substituent.

Examples of the halogen atom are fluorine, chlorine, bromine and iodine. In addition, examples of the substituted or unsubstituted amino group include an alkylamino group, an arylamino group, and an aralkylamino group.

The alkoxycarbonyl group is represented by -COOY'. Examples of Y' are the same as the examples of the alkyl group. The alkylamino group and the aralkylamino group are represented by -NQ¹Q². Examples for each of Q¹ and Q² are the same as the examples described in relation to the alkyl group and the aralkyl group (i.e., a group obtained by substituting a hydrogen atom of an alkyl group with an aryl group), and preferable examples for each of Q¹ and Q² are also the same as those described in relation to the alkyl group and the aralkyl group. One of Q¹ and Q² may be a hydrogen atom. Note that the aralkyl group is a group obtained by substituting the hydrogen atom of the alkyl group with the aryl group.

The arylamino group is represented by -NAr¹Ar². Examples for each of Ar¹ and Ar² are the same as the examples described in relation to the non-fused aromatic hydrocarbon group.

One of Ar¹ and Ar² may be a hydrogen atom.

M represents aluminum (Al), gallium (Ga) or indium (In), among which In is preferable.

L in the formula (B1) represents a group represented by a formula (B2) or (B3) below.

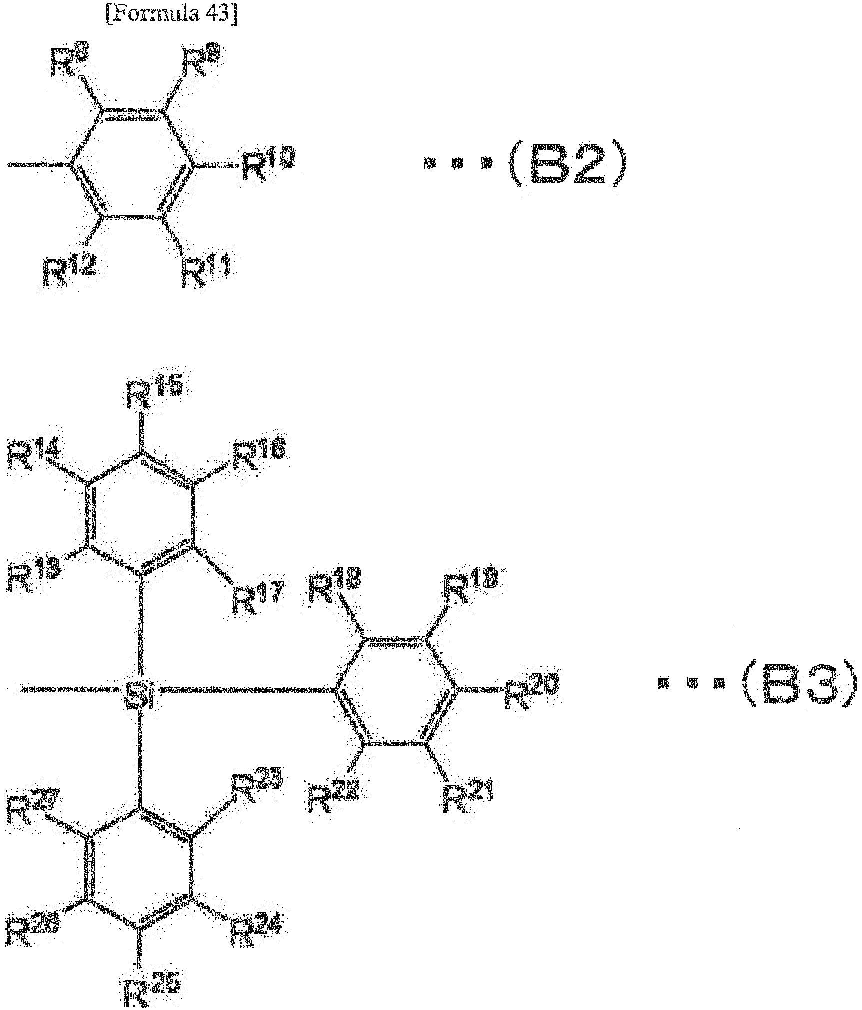

In the formula (B2), R⁸ to R¹² independently represent a hydrogen atom or a hydrocarbon group having 1 to 40 carbon atoms. Adjacent ones of the hydrocarbon groups may form a cyclic structure. The hydrocarbon group may have a substituent.

In the formula (B3), R¹³ to R²⁷ independently represent a hydrogen atom or a hydrocarbon group having 1 to 40 carbon atoms.
Adjacent ones of the hydrocarbon groups may form a cyclic structure. The hydrocarbon group may have a substituent.

Examples of the hydrocarbon group having 1 to 40 carbon atoms represented by each of R⁸ to R¹² and R¹³ to R²⁷ in the formulae (B2) and (B3) are the same as those of R² to R⁷ in the formula (B1).

Examples of a divalent group formed when adjacent groups of R⁸ to R¹² and adjacent groups of R¹³ to R²⁷ form a cyclic structure are a tetramethylene group, pentamethylene group, hexamethylene group, diphenylinethane-2,2'-diyl group, diphenylethane-3,3'-diyl group and diphenylpropane-4,4'-diyl group.

The electron transporting layer preferably contains at least one of nitrogen-containing heterocyclic derivatives respectively represented by the following formulae (B4) to (B6).

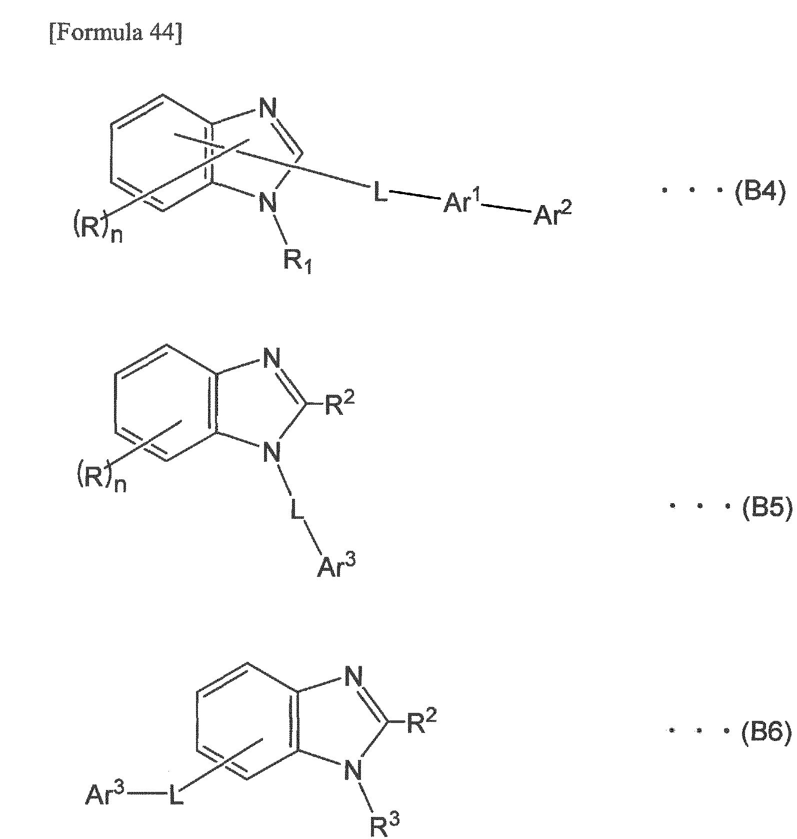

In the formulae (B4) to (B6), R represents a hydrogen atom, an aromatic hydrocarbon group having 6 to 60 ring carbon atoms, a pyridyl group, a quinolyl group, an alkyl group having 1 to 20 carbon atoms, or an alkoxy group having 1 to 20 carbon atoms n is an integer in a range of 0 to 4.

In the formulae (B4) to (B6), R¹ represents an aromatic hydrocarbon group having 6 to 60 ring carbon atoms, a pyridyl group, a quinolyl group, an alkyl group having 1 to 20 carbon atoms, or an alkoxy group having 1 to 20 carbon atoms.

In the formulae (B4) to (B6), R² and R³ represent a hydrogen atom, an aromatic hydrocarbon group having 6 to 60 ring carbon atoms, a pyridyl group, a quinolyl group, an alkyl group having 1 to 20 carbon atoms, or an alkoxy group having 1 to 20 carbon atoms.

In the formulae (B4) to (B6), L represents an aromatic hydrocarbon group having 6 to 60 ring carbon atoms, a pyridinylene group, a quinolinylene group, or a fluorenylene group.

In the formulae (B4) to (B6), Ar¹ represents an aromatic hydrocarbon group having 6 to 60 ring carbon atoms, a pyridinylene group, or a quinolinylene group.

In the formulae (B4) to (B6), Ar² represents an aromatic hydrocarbon group having 6 to 60 ring carbon atoms, a pyridyl group, a quinolyl group, an alkyl group having 1 to 20 carbon atoms, or an alkoxy group having 1 to 20 carbon atoms.

In the formulae (B4) to (B6), Ar³ represents an aromatic hydrocarbon group having 6 to 60 ring carbon atoms, a pyridyl group, a quinolyl group, an alkyl group having 1 to 20 carbon atoms, an alkoxy group having 1 to 20 carbon atoms, or a group represented by -Ar¹-Ar² in which Ar¹ and Ar² are the same as the above.

The aromatic hydrocarbon group, pyridyl group, quinolyl group, alkyl group, alkoxy group, pyridinylene group, quinolinylene group and fluorenylene group which are described in relation to R, R¹, R², R³, L, Ar¹, Ar² and Ar³ in the formulae (B4) to (B6) may have a substituent.

As an electron transporting compound for the electron injecting layer or the electron transporting layer, 8-hydroxyquinoline or a metal complex of its derivative, an oxadiazole derivative and a nitrogen-containing heterocyclic derivative are preferable. An example of the 8-hydroxyquinoline or the metal complex of its derivative is a metal chelate oxinoid compound containing a chelate of oxine (typically 8-quinolinol or 8-hydroxyquinoline). For instance, tris(8-quinolinol) aluminum can be used. Examples of the oxadiazole derivative are as follows.

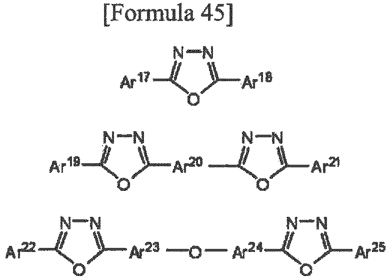

In each of the formulae of the oxadiazole derivatives, Ar¹⁷, Ar¹⁸, Ar¹⁹, Ar²¹, Ar²² and Ar²⁵ represent an aromatic hydrocarbon group having 6 to 40 ring carbon atoms.

Note that the aromatic hydrocarbon group described herein may have a substituent. Ar¹⁷, Ar¹⁹ and Ar²² are respectively the same as or different from Ar¹⁸, Ar²¹ and Ar²⁵.

Examples of the aromatic hydrocarbon group described herein are a phenyl group, naphthyl group, biphenyl group, anthranil group, perylenyl group and pyrenyl group. Examples of the substituent therefor are an alkyl group having 1 to 10 carbon atoms, alkoxy group having 1 to 10 carbon atoms and cyano group.

In each of the formulae of the oxadiazole derivatives, Ar²⁰, Ar²³ and Ar²⁴ are a divalent aromatic hydrocarbon group having 6 to 40 ring carbon atoms.

Note that the aromatic hydrocarbon group described herein may have a substituent.

Ar²³ and Ar²⁴ are the same or different.

Examples of the divalent aromatic hydrocarbon group described herein are a phenylene group, naphthylene group, biphenylene group, anthranylene group, perylenylene group and pyrenylene group. Examples of the substituent therefor are an alkyl group having 1 to 10 carbon atoms, alkoxy group having 1 to 10 carbon atoms and cyano group.

Such an electron transport compound is preferably an electron transport compound that can be favorably formed into a thin film(s). Examples of the electron transporting compounds are as follows.

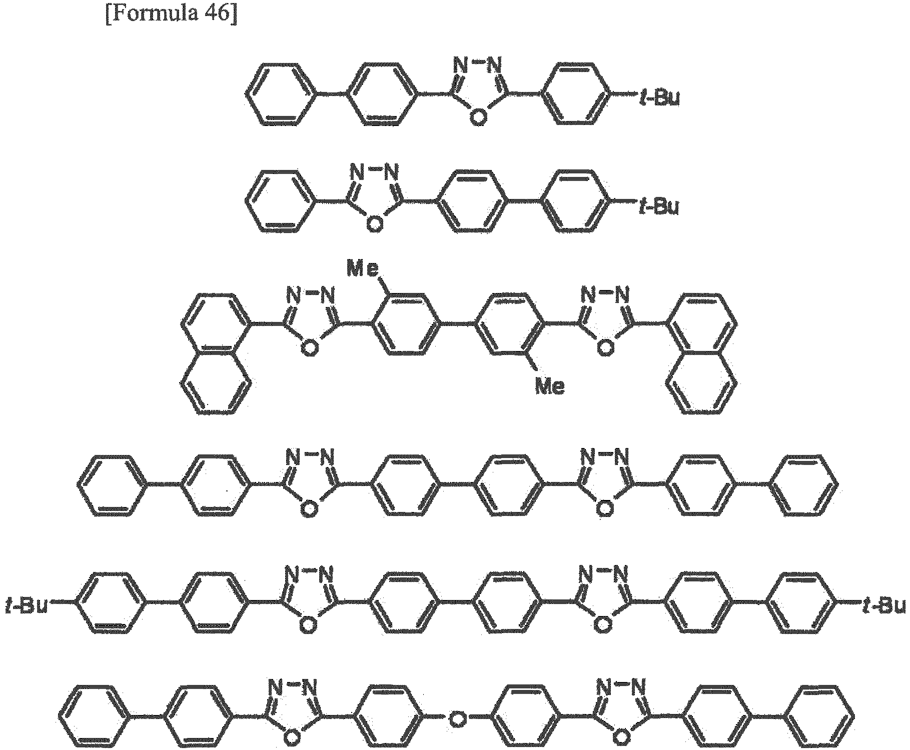

An example of the nitrogen-containing heterocyclic derivative as the electron transporting compound is a nitrogen-containing compound that is not a metal complex, the derivative being formed of an organic compound represented by one of the following formulae. Examples of the nitrogen-containing heterocyclic derivative are a five-membered ring or six-membered ring derivative having a skeleton represented by the following formula (B7) and a derivative having a structure represented by the following formula (B8).

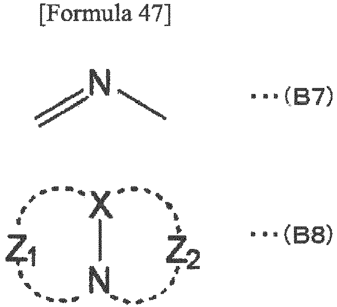

In the formula (B8), X represents a carbon atom or a nitrogen atom. Z₁ and Z₂ each independently represent a group of atoms capable of forming a nitrogen-containing heterocycle.

More preferably, the nitrogen-containing heterocyclic derivative is an organic compound having nitrogen-containing aromatic polycyclic series having a five-membered ring or six-membered ring. Moreover, when the nitrogen-containing heterocyclic derivative includes such nitrogen-containing aromatic polycyclic series having plural nitrogen atoms, the nitrogen-containing heterocyclic derivative is preferably a nitrogen-containing aromatic polycyclic organic compound having a skeleton formed by a combination of the skeletons respectively represented by the formulae (B7) and (B8), or by a combination of the skeletons respectively represented by the formulae (B7) and (B9).

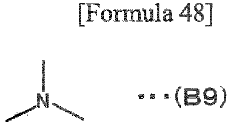

A nitrogen-containing group of the nitrogen-containing aromatic polycyclic organic compound is selected from nitrogen-containing heterocyclic groups respectively represented by the following formulae.

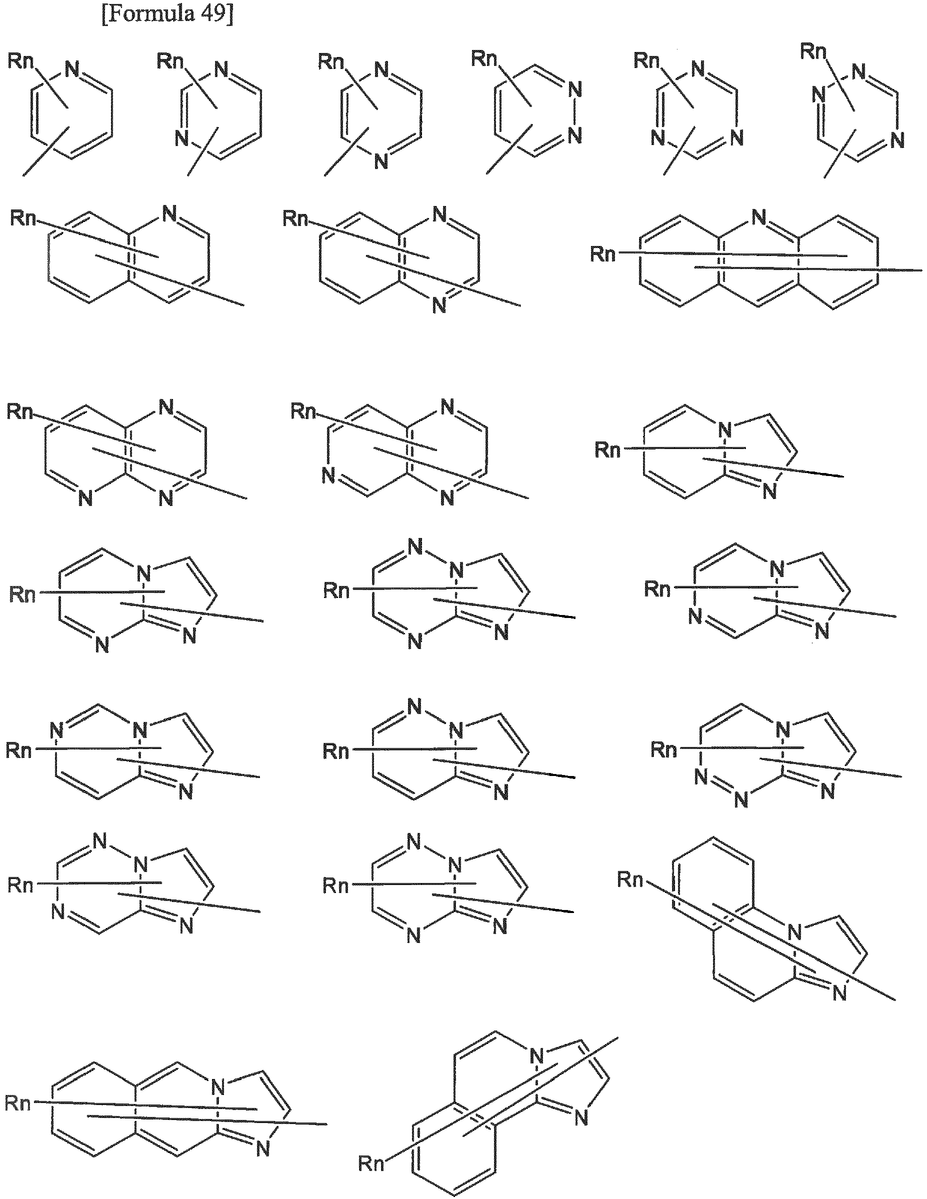

In each of the formulae of the nitrogen-containing heterocyclic groups, R represents an aromatic hydrocarbon group having 6 to 40 ring carbon atoms, an aromatic heterocyclic group having 2 to 40 ring carbon atoms, an alkyl group having 1 to 20 carbon atoms, or an alkoxy group having 1 to 20 carbon atoms.

In each of the formulae of the nitrogen-containing heterocyclic groups, n is an integer of 0 to 5. When n is 2 or more, a plurality of R may be mutually the same or different.

A preferable specific compound is a nitrogen-containing heterocyclic derivative represented by the following formula (B 10).

HAr-L¹-Ar¹-Ar² (B10)

In the formula (B10), HAr represents a nitrogen-containing heterocyclic group having 1 to 40 ring carbon atoms.

In the formula (B10), L¹ represents a single bond, an aromatic hydrocarbon group having 6 to 40 ring carbon atoms, or an aromatic heterocyclic group having 2 to 40 ring carbon atoms.

In the formula (B10), Ar¹ is a divalent aromatic hydrocarbon group having 6 to 40 ring carbon atoms. In the formula (B10), Ar² represents an aromatic hydrocarbon group having 6 to 40 ring carbon atoms, or an aromatic heterocyclic group having 2 to 40 ring carbon atoms.

The nitrogen-containing heterocyclic group, aromatic hydrocarbon group and aromatic heterocyclic group described in relation to HAr, L¹, Ar¹ and Ar² in the formula (B10) may have a substituent.

HAr in the formula (B10) is exemplarily selected from the following group.

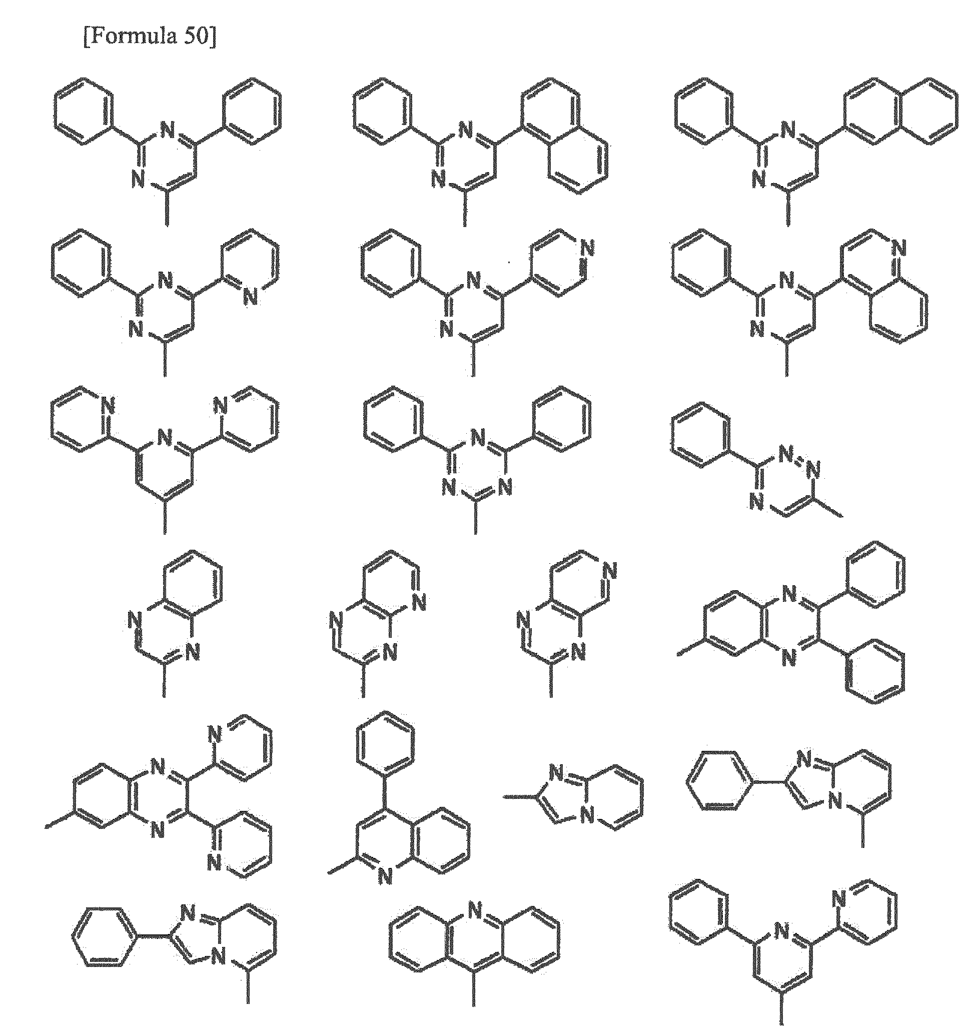

L¹ in the formula (B10) is exemplarily selected from the following group.

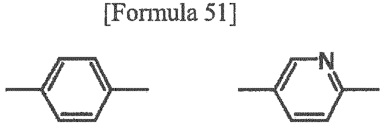

Ar¹ in the formula (B10) is exemplarily selected from the following arylanthranil group.

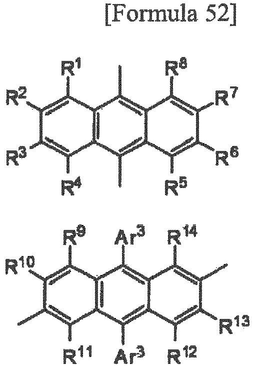

In the formula of the arylanthranil group, R¹ to R¹⁴ independently represent a hydrogen atom, a halogen atom, an alkyl group having 1 to 20 carbon atoms, an alkoxy group having 1 to 20 carbon atoms, an aryloxy group having 6 to 40 ring carbon atoms, an aromatic hydrocarbon group having 6 to 40 ring carbon atoms, or an aromatic heterocyclic group having 2 to 40 ring carbon atoms.

In the arylanthranil group, Ar³ represents an aromatic hydrocarbon group having 6 to 40 ring carbon atoms, or an aromatic heterocyclic group having 2 to 40 ring carbon atoms.

The aromatic hydrocarbon group and aromatic heterocyclic group described in relation to R¹ to R¹⁴ and Ar³ in the formula of the arylanthranil may have a substituent.

All of R¹ to R⁸ of a nitrogen-containing heterocyclic derivative may be hydrogen atoms.

In the formula of the arylanthranil group, Ar² is exemplarily selected from the following group.

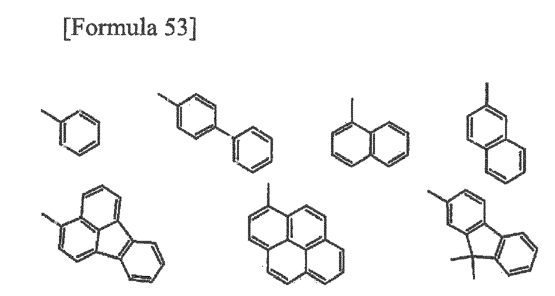

Other than the above, the following compound (see JP-A-9-3448) can be favorably used for the nitrogen-containing aromatic polycyclic organic compound as the electron transporting compound.

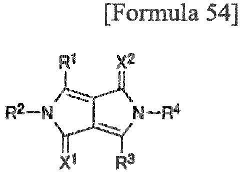

In the formula of the nitrogen-containing aromatic polycyclic organic compound, R¹ to R⁴ independently represent a hydrogen atom, an aliphatic group, an alicyclic group, a carbocyclic aromatic cyclic group, or a heterocyclic group. Note that the aliphatic group, alicyclic group, carbocyclic aromatic cyclic group and heterocyclic group may have a substituent.

In the formula of the nitrogen-containing aromatic polycyclic organic compound, X¹ and X² each independently represent an oxygen atom, sulfur atom or dicyanomethylene group.

The following compound (see JP-A-2000-173774) can also be favorably used for the electron transporting compound.

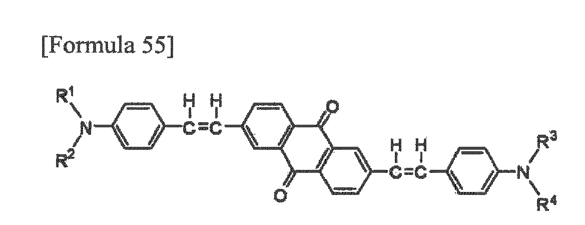

In the formula, R¹, R², R³ and R⁴, which may be mutually the same or different, each represent an aromatic hydrocarbon group or a fused aromatic hydrocarbon group represented by the following formula.

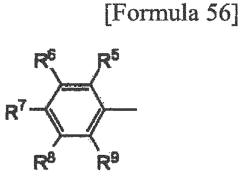

In the formula, R⁵, R⁶, R⁷, R⁸ and R⁹, which may be mutually the same or different, each represent a hydrogen atom, a saturated or unsaturated alkoxy group, alkyl group, amino group or alkylamino group. At least one of R⁵, R⁶, R⁷, R⁸ and R⁹ represents a saturated or unsaturated alkoxy group, alkyl group, amino group or alkylamino group.

A polymer compound containing the nitrogen-containing heterocyclic group or a nitrogen-containing heterocyclic derivative may be used for the electron transporting compound.

Although a thickness of the electron injecting layer or the electron transporting layer is not particularly limited, the thickness is preferably in a range of 1 nm to 100 nm.

The electron injecting layer preferably contains an inorganic compound such as an insulator or a semiconductor in addition to the nitrogen-containing cyclic derivative. Such an insulator or a semiconductor, when contained in the electron injecting layer, can effectively prevent a current leak, thereby enhancing electron capability of the electron injecting layer.

For such an insulator, at least one metal compound selected from a group of alkali metal chalcogenide, alkaline-earth metal chalcogenide, halogenide of alkali metal, and halogenide of alkaline-earth metal may preferably be utilized. A configuration in which the electron injecting layer is formed by these alkali metal chalcogenide and the like is advantageous in that the electron injecting property is further improved. Specifically, preferable examples of the alkali metal chalcogenide are lithium oxide (Li₂O), potassium oxide (K₂O), sodium sulfide (Na₂S), sodium selenide (Na₂Se) and sodium oxide (Na₂O). Preferable examples of the alkaline-earth metal chalcogenide are calcium oxide (CaO), barium oxide (BaO), strontium oxide (SrO), beryllium oxide (BeO), barium sulfide (BaS) and calcium selenide (CaSe). Preferable examples of the halogenide of the alkali metal are lithium fluoride (LiF), sodium fluoride (NaF), potassium fluoride (KF), lithium chloride (LiCI), potassium chloride (KCl) and sodium chloride (NaCl). Preferable examples of the halogenide of the alkaline-earth metal are fluorides such as calcium fluoride (CaF₂), barium fluoride (BaF₂), strontium fluoride (SrF₂), magnesium fluoride (MgF₂) and beryllium fluoride (BeF₂), and halogenides other than the fluorides.

Examples of the semiconductor are one of or a combination of two or more of an oxide, a nitride or an oxidized nitride containing at least one element selected from barium (Ba), calcium (Ca), strontium (Sr), ytterbium (Yb), aluminum (Al), gallium (Ga), indium (In), lithium (Li), sodium (Na), cadmium (Cd), magnesium (Mg), silicon (Si), tantalum (Ta), antimony (Sb) and zinc (Zn). An inorganic compound for forming the electron injecting layer is preferably a microcrystalline or amorphous insulative thin-film. When the electron injecting layer is formed of such an insulative thin-film, more uniform thin-film can be formed, thereby reducing pixel defects such as a dark spot. Examples of such an inorganic compound are the above-described alkali metal chalcogenide, alkaline-earth metal chalcogenide, halogenide of the alkali metal and halogenide of the alkaline-earth metal.

When the electron injecting layer contains such an insulator or a semiconductor, a thickness thereof is preferably in a range of approximately 0.1 nm to 15 nm. The electron injecting layer in this exemplary embodiment may preferably contain the above-described reduction-causing dopant.

### Electron-donating Dopant and Organic Metal Complex

In the organic EL device according to this exemplary embodiment, at least one of an electron-donating dopant and an organic metal complex is preferably contained in an interfacial region between the cathode and the organic thin-film layer.

With this arrangement, the organic EL device can emit light with enhanced luminance intensity and have a longer lifetime.

The electron-donating dopant may be at least one selected from an alkali metal, an alkali metal compound, an alkaline-earth metal, an alkaline-earth metal compound, a rare-earth metal, a rare-earth metal compound and the like.

The organic metal complex may be at least one selected from an organic metal complex including an alkali metal, an organic metal complex including an alkaline-earth metal, an organic metal complex including a rare-earth metal and the like.

Examples of the alkali metal are lithium (Li) (work function: 2.93eV), sodium (Na) (work function: 2.36 eV), potassium (K) (work function: 2.28 eV), rubidium (Rb) (work function: 2.16 eV) and cesium (Cs) (work function: 1.95 eV), which particularly preferably has a work function of 2.9 eV or less. Among the above, the alkali metal is preferably K, Rb or Cs, more preferably Rb or Cs, the most preferably Cs.

Examples of the alkaline-earth metal are calcium (Ca) (work function: 2.9 eV), strontium (Sr) (work function: 2.0 to 2.5 eV), and barium (Ba) (work function: 2.52 eV), among which a substance having a work function of 2.9 eV or less is particularly preferable.

Examples of the rare-earth metal are scandium (Sc), yttrium (Y), cerium (Ce), terbium (Tb), and ytterbium (Yb), among which a substance having a work function of 2.9 eV or less is particularly preferable.

Since the above preferred metals have particularly high reducibility, addition of a relatively small amount of the metals to an electron injecting zone can enhance luminance intensity and lifetime of the organic EL device.

Examples of the alkali metal compound are an alkali oxide such as lithium oxide (Li₂O), cesium oxide (Cs₂O) and potassium oxide (K₂O), and an alkali halogenide such as sodium fluoride (NaF), cesium fluoride (CsF) and potassium fluoride (KF), among which lithium fluoride (LiF), lithium oxide (Li₂O) and sodium fluoride (NaF) are preferable.

Examples of the alkaline-earth metal compound are barium oxide (BaO), strontium oxide (SrO), calcium oxide (CaO) and a mixture thereof, i.e., barium strontium oxide (BaₓSr₁₋ₓO) (0<x<1), barium calcium oxide (BaₓCa₁₋ₓO) (0<x<1), among which BaO, SrO and CaO are preferable.

Examples of the rare earth metal compound are ytterbium fluoride (YbF₃), scandium fluoride (ScF₃), scandium oxide (ScO₃), yttrium oxide (Y₂O₃), cerium oxide (Ce₂O₃), gadolinium fluoride (GdF₃) and terbium fluoride (TbF₃), among which YbF₃, ScF₃, and TbF₃ are preferable.

The organic metal complex is not specifically limited as long as containing at least one metal ion of an alkali metal ion, an alkaline-earth metal ion and a rare earth metal ion. A ligand for each of the complexes is preferably quinolinol, benzoquinolinol, acridinol, phenanthridinol, hydroxyphenyl oxazole, hydroxyphenyl thiazole, hydroxydiaryl oxadiazole, hydroxydiaryl thiadiazole, hydroxyphenyl pyridine, hydroxyphenyl benzoimidazole, hydroxybenzo triazole, hydroxy fluborane, bipyridyl, phenanthroline, phthalocyanine, porphyrin, cyclopentadiene, β-diketones, azomethines, or a derivative thereof, but the ligand is not limited thereto.

The electron-donating dopant and the organic metal complex are added to preferably form a layer or an island pattern in the interfacial region. The layer or the island pattern of the electron-donating dopant and the organic metal complex is preferably formed by evaporating at least one of the electron-donating dopant and the organic metal complex by resistance heating evaporation while an emitting material for forming the interfacial region or an organic substance as an electron-injecting material are simultaneously vapor-deposited, so that at least one of the electron-donating dopant and an organic metal complex reduction-causing dopant is dispersed in the organic substance. Dispersion concentration at which the electron-donating dopant is dispersed in the organic substance is a mole ratio (the organic substance to the electron-donating dopant or the organic metal complex) of 100:1 to 1:100, preferably 5:1 to 1:5.

When at least one of the electron-donating dopant and the organic metal complex forms a layer, the emitting material or the electron injecting material for forming the organic layer of the interfacial region is initially layered, and then, at least one of the electron-donating dopant and the organic metal complex is singularly vapor-deposited thereon by resistance heating evaporation to preferably form a 0.1 nm- to 15 nm-thick layer.

When at least one of the electron-donating dopant and the organic metal complex forms an island pattern, the emitting material or the electron injecting material for forming the organic layer of the interfacial region is initially layered, and then, at least one of the electron-donating dopant is singularly vapor-deposited thereon by resistance heating evaporation to preferably form a 0.05 nm- to 1 nm-thick island pattern.

A ratio of the main component to at least one of the electron-donating dopant and the organic metal complex in the organic EL device according to the exemplary embodiment is preferably a mole ratio (the main component to the electron-donating dopant or the organic metal complex) of 5:1 to 1:5, more preferably 2:1 to 1:2.

### Formation Method of Each Layer of Organic EL Device

A method of forming each of the layers in the organic EL device according to this exemplary embodiment is not particularly limited. Conventionally-known methods such as vacuum deposition and spin coating may be employed for forming the layers. The organic compound layer containing the compound represented by the formula (1), which is used in the organic EL device according to this exemplary embodiment, may be formed by a conventional coating method such as vacuum deposition, molecular beam epitaxy (MBE method) and coating methods using a solution such as a dipping, spin coating, casting, bar coating, and roll coating.

### Thickness of Each Layer of Organic EL Device

A thickness of the emitting layer is preferably in the range of 5 nm to 50 nm, more preferably in the range of 7 nm to 50 nm and most preferably in the range of 10 nm to 50 nm. By forming the emitting layer at the thickness of 5 nm or more, the emitting layer is easily formable and chromaticity is easily adjustable. By forming the emitting layer at the thickness of 50 nm or less, increase in the drive voltage is suppressible.

A thickness of the organic compound layer other than the emitting layer is not particularly limited, but is preferably in a typical range of several nm to 1 µm. When the thickness is provided in the above range, defects such as pin holes caused by an excessively thinned film can be avoided while increase in the drive voltage caused by an excessively thickened film can be suppressed to prevent deterioration of the efficiency.

### Measurement Method of Triplet Energy

Triplet energy EgT of each of the compounds was measured by the following method.

Each of the compounds was measured by a known method of measuring phosphorescence (e.g. a method described in "Hikarikagaku no Sekai (The World of Photochemistry)" (edited by The Chemical Society of Japan, 1993, on and near page 50). Specifically, each of the compounds was dissolved in a solvent (sample: 10 µmol/L, EPA (diethylether:isopentane:ethanol = 5:5:5 in volume ratio, each solvent in a spectroscopic grade), thereby forming a sample for phosphorescence measurement. The sample for phosphorescence measurement was put into a quartz cell, cooled to 77(K) and irradiated with excitation light, so that phosphorescence intensity was measured while changing a wavelength. The phosphorescence spectrum was expressed in coordinates of which ordinate axis indicated phosphorescence intensity and of which abscissa axis indicated the wavelength.

A tangent was drawn to the rise of the phosphorescent spectrum on the short-wavelength side, and a wavelength value λedge (nm) at an intersection of the tangent and the abscissa axis was obtained. The wavelength value was converted to an energy value by the following conversion equation. The energy value was defined as EgT.

### The conversion equation: EgT (eV)=1239.85/λedge

For phosphorescence measurement, a spectrophotofluorometer body F-4500 and optional accessories for low temperature measurement (which were manufactured by Hitachi High-Technologies Corporation) were used. The measurement instrument is not limited to this arrangement. A combination of a cooling unit, a low temperature container, an excitation light source and a light-receiving unit may be used for measurement.

### Modifications of Exemplary Embodiment(s)

It should be noted that the invention is not limited to the above exemplary embodiment but may include any modification and improvement as long as such modification and improvement are compatible with the invention.

An arrangement of the organic EL device is not particularly limited to the arrangement of the organic EL device 1 shown in Fig. 1. For instance, an electron blocking layer may be provided to the emitting layer adjacent to the anode while a hole blocking layer may be provided to the emitting layer adjacent to the cathode. With this arrangement, the electrons and the holes can be confined in the emitting layer, thereby enhancing probability of exciton generation in the emitting layer.

The emitting layer is not limited to a single layer, but may be provided as laminate by a plurality of emitting layers. When the organic EL device has the plurality of emitting layers, at least one of the emitting layers preferably contains a biscarbazole derivative of the invention.

Moreover, when the organic EL device includes the plurality of emitting layers, the plurality of emitting layers may be adjacent to each other, or may be laminated on each other via a layer other than the emitting layers (e.g., a charge generating layer). Examples

Next, the invention will be described in more detail by exemplifying Example(s) and Comparative(s). However, the invention is not limited by the description of Example(s).

### Synthesis of Compound(s)

### Synthesis Example 1 (Synthesis of Compound 1)

A synthesis scheme of the compound 1 is shown below.

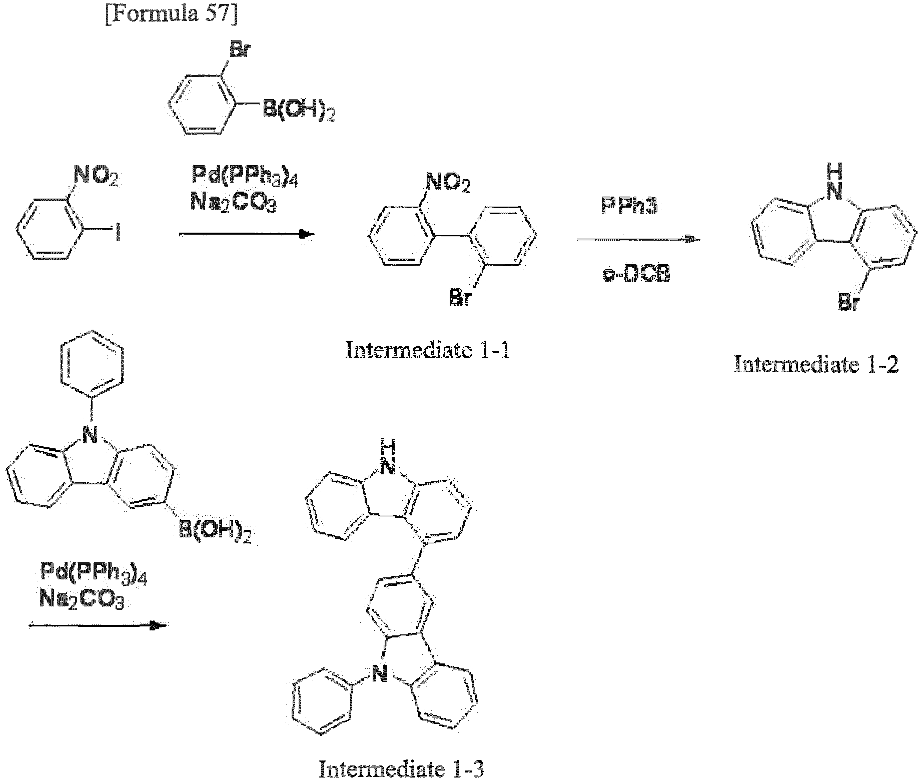

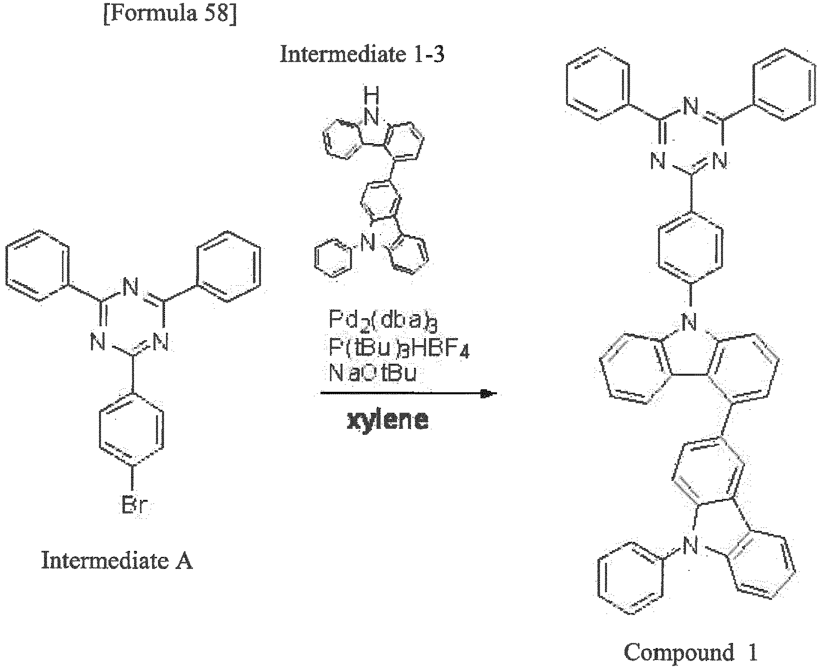

In synthesizing the compound 1, initially, an intermediate 1-1 was synthesized as follows.

Under argon atmosphere, a mixture of 25g (100 mmol) of o-iodenitrobenzene, 21g (105 mmol) of o-bromophenyl boronic acid, 2.3 g (2 mmol) of tetrakis(triphenylphosphine)palladium(0), 150 mL of toluene, 150 mL of xyethane, and 150 mL of an aqueous solution of 2M sodium carbonate was stirred at 80 degrees C for eight hours. After the organic phase was separated and the solvent was vapor-deposited by an evaporator, the obtained residue was purified by silica-gel column chromatography, so that the intermediate 1-1 (20 g, a yield of 72%) was obtained.

Subsequently, an intermediate 1-2 was synthesized in the following manner.

Under argon atmosphere, a mixture of 20g (72 mmol) of the intermediate 1-1, 18.9 g (72 mmol) of triphenylphosphine, and 100 mL of o-dichlorobenzene was heated at 180 degrees C for eight hours while being stirred. Water was added to the reaction solution to precipitate solid. Then, the obtained solid was filtrated. The obtained solid was purified by silica-gel column chromatography, so that the intermediate 1-2 (8.4 g, a yield of 47%) was obtained.

Subsequently, an intermediate 1-3 was synthesized in the following manner.

Under argon atmosphere, a mixture of 7.4 g (30 mmol) of the intermediate 1-2, 8.7 g (30 mmol) of 9-phenylcarbazol-3-boronic acid, 0.69 g (0.6 mmol) of tetrakis(triphenylphosphine)palladium(0), 45 mL of toluene, 45 mL of dimethoxyethane, and 45 mL of an aqueous solution of 2M sodium carbonate was stirred at 80 degrees C for eight hours. After the organic phase was separated and the solvent was vapor-deposited by an evaporator, the obtained residue was purified by silica-gel column chromatography, so that the intermediate 1-3 (9.1 g, a yield of 74%) was obtained.

Subsequently, a compound 1 was synthesized in the following manner.

Under argon atmosphere, a mixture of 2.1 g (5.5 mmol) of an intermediate A, 2.0 g (5 mmol) of the intermediate 1-3, 0.09 g (0.1 mmol) of tris(dibenzylideneacetone)dipalladium(0), 0.11 g (0.4 mmol) of tri-tert-butylphosphonium tetrafluoroborate, 0.67 g (7 mmol) of sodium tert-butoxide, and 20 mL of xylene was heated for eight hours while being refluxed. Water was added to the mixture and the obtained mixture was stirred for one hour. After the formed solid was filtrated and washed with water and methanol, the obtained solid was purified by silica-gel column chromatography, so that the compound 1 (3.0 g, a yield of 85%) was obtained.

An analysis result by FD-MS (Field Desorption ionization-Mass Spectrometry) is shown below.
FD-MS:calcd for C₅₁H₃₃N₅=715.27,
found m/z=715(M⁺,100)

### Synthesis Example 2 (Synthesis of Compound 2)

A synthesis scheme of the compound 2 is shown below.

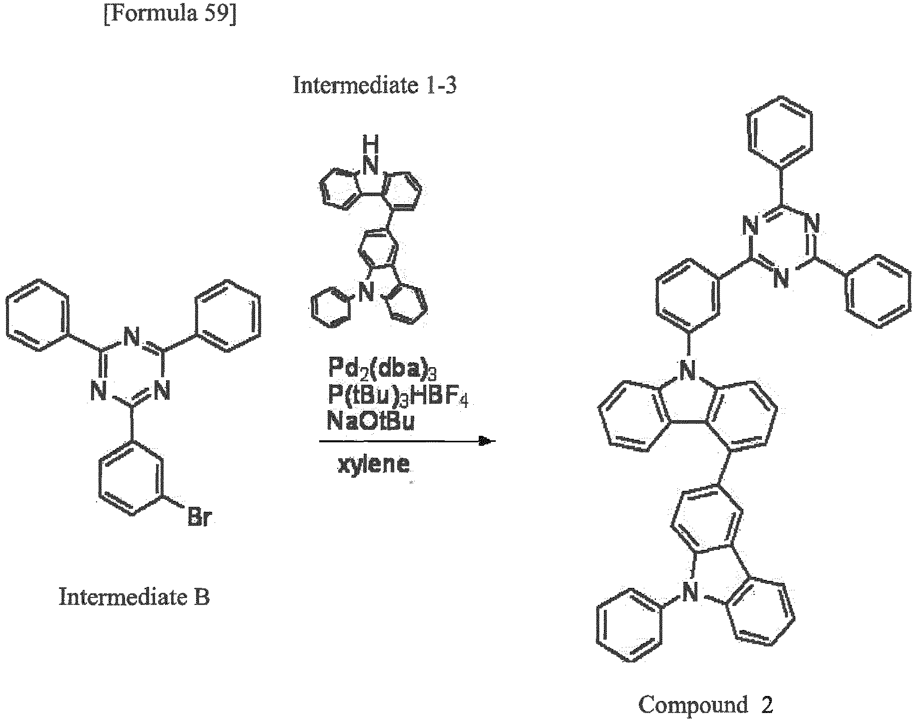

The compound 2 was synthesized in the following manner.

Under argon atmosphere, a mixture of 2.1 g (5.5 mmol) of an intermediate B, 2.0 g (5 mmol) of the intermediate 1-3, 0.09 g (0.1 mmol) of tris(dibenzylideneacetone)dipalladium(0), 0.11 g (0.4 mmol) of tri-tert-butylphosphonium tetrafluoroborate, 0.67 g (7 mmol) of sodium tert-butoxide, and 20 mL of xylene was heated for eight hours while being refluxed. Water was added to the mixture and the obtained mixture was stirred for one hour. After the formed solid was filtrated and washed with water and methanol, the obtained solid was purified by silica-gel column chromatography, so that the compound 1-2 (1.9 g, a yield of 53%) was obtained.

An analysis result by FD-MS (Field Desorption ionization-Mass Spectrometry) is shown below.
FD-MS:calcd for C₅₁H₃₃N₅=715.27,
found m/z=715(M⁺,100)

### Synthesis Example 3 (Synthesis of Compound 3)

A synthesis scheme of a compound 3 is shown below.

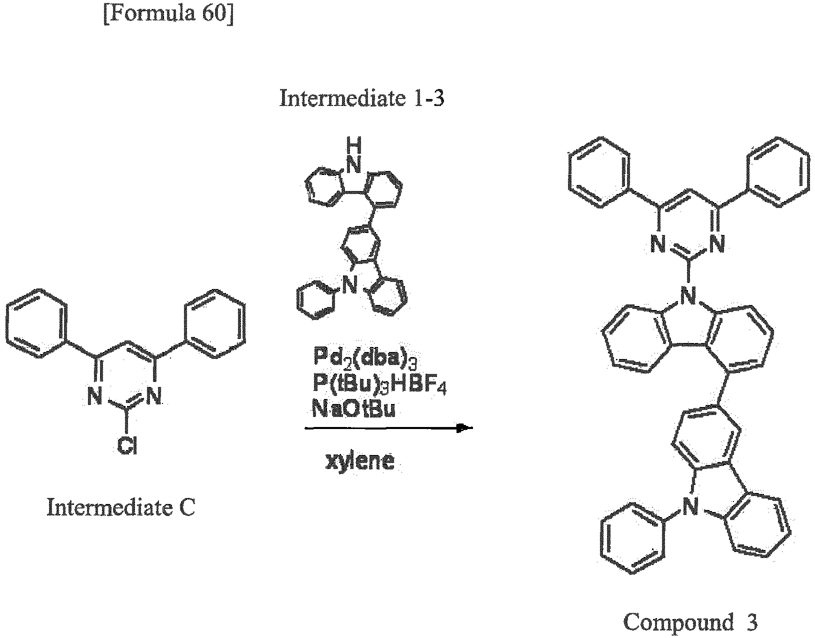

The compound 3 was synthesized in the following manner.

Under argon atmosphere, a mixture of 1.46 g (5.5 mmol) of an intermediate C, 2.0 g (5 mmol) of the intermediate 1-3, 0.09 g (0.1 mmol) of tris(dibenzylideneacetone)dipalladium(0), 0.11 g (0.4 mmol) of tri-tert-butylphosphonium tetrafluoroborate, 0.67 g (7 mmol) of sodium tert-butoxide, and 20 mL of xylene was heated for eight hours while being refluxed. Water was added to the mixture and the obtained mixture was stirred for one hour. After the formed solid was filtrated and washed with water and methanol, the obtained solid was purified by silica-gel column chromatography, so that the compound 3 (3.0 g, a yield of 85%) was obtained.

An analysis result by FD-MS (Field Desorption ionization-Mass Spectrometry) is shown below.
FD-MS:calcd for C₄₆H₃₀N₄=638.25,
found m/z=63 8(M⁺,100)

### Synthesis Example 4 (Synthesis of Compound 4)

A synthesis scheme of a compound 4 is shown below.

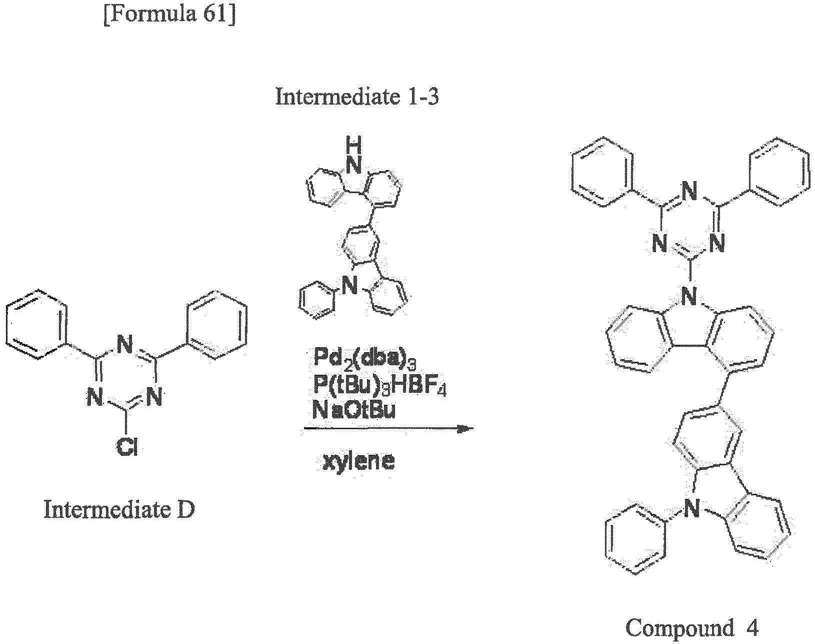

The compound 4 was synthesized in the following manner.

Under argon atmosphere, a mixture of 1.47 g (5.5 mmol) of an intermediate D, 2.0 g (5 mmol) of the intermediate 1-3, 0.09 g (0.1 mmol) of tris(dibenzylideneacetone)dipalladium(0), 0.11 g (0.4 mmol) of tri-tert-butylphosphonium tetrafluoroborate, 0.67 g (7 mmol) of sodium tert-butoxide, and 20 mL of xylene was heated for eight hours while being refluxed. Water was added to the mixture and the obtained mixture was stirred for one hour. After the formed solid was filtrated and washed with water and methanol, the obtained solid was purified by silica-gel column chromatography, so that the compound 4 (2.2 g, a yield of 69%) was obtained.

An analysis result by FD-MS (Field Desorption ionization-Mass Spectrometry) is shown below.
FD-MS:calcd for C₄₆H₃₀N₄=639.24,
found m/z=639(M⁺,100)

### Measurement of Triplet Energy

Triplet energy (EgT) of each of the compounds 1 to 4 synthesized in Synthesis Examples 1 to 4 is shown in Table 1. A compound to be compared with the compounds 1 to 4 is exemplified by a biscarbazole derivative in which carbazolyl groups are bonded to each other at their positions 3 (hereinafter, referred to as a 3,3-bonded biscarbazole derivative: see the following compounds a, b and c). Triplet energy (EgT) of each of the biscarbazole derivatives is also shown in Table 1.

**Table 1**

| | EgT (eV) |
|---|---|
| Compound 1 | 2.84 |
| Compound 2 | 2.91 |
| Compound 3 | 2.93 |
| Compound 4 | 3.02 |
| Compound a | 2.77 |
| Compound b | 2.84 |
| Compound c | 2.91 |

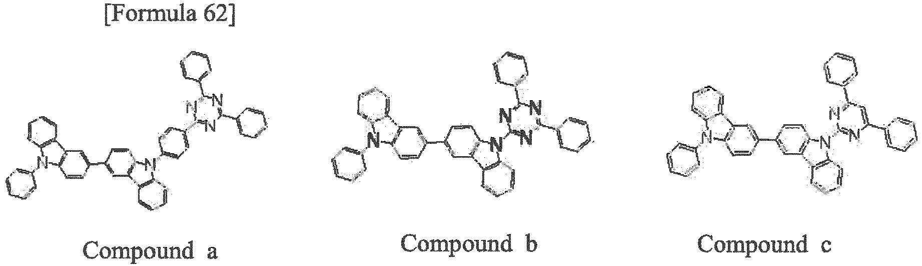

Each of the compounds 1 to 4 is a biscarbazole derivative in which one carbazolyl group is bonded at its position 4 to a position 3 of the other carbazolyl group (hereinafter, referred to as a 4,3-bonded biscarbazole derivative). It has been found that the 4,3-bonded biscarbazole derivative such as the compounds 1 to 4 has a relatively larger triplet energy than the 3,3-bonded biscarbazole derivative such as the compounds a to c. Accordingly, it has been found that the 4,3-bonded biscarbazole derivative is useful as a material not only for a green-phosphorescent organic EL device but also for a blue-phosphorescent organic EL device.

### Preparation and Evaluation of Organic EL Device

The organic EL devices were prepared in the following manner and evaluated.

Compounds used for preparing the organic EL device are as follows in addition to the compounds 1 to 4 described in Synthesis Examples 1 to 4.

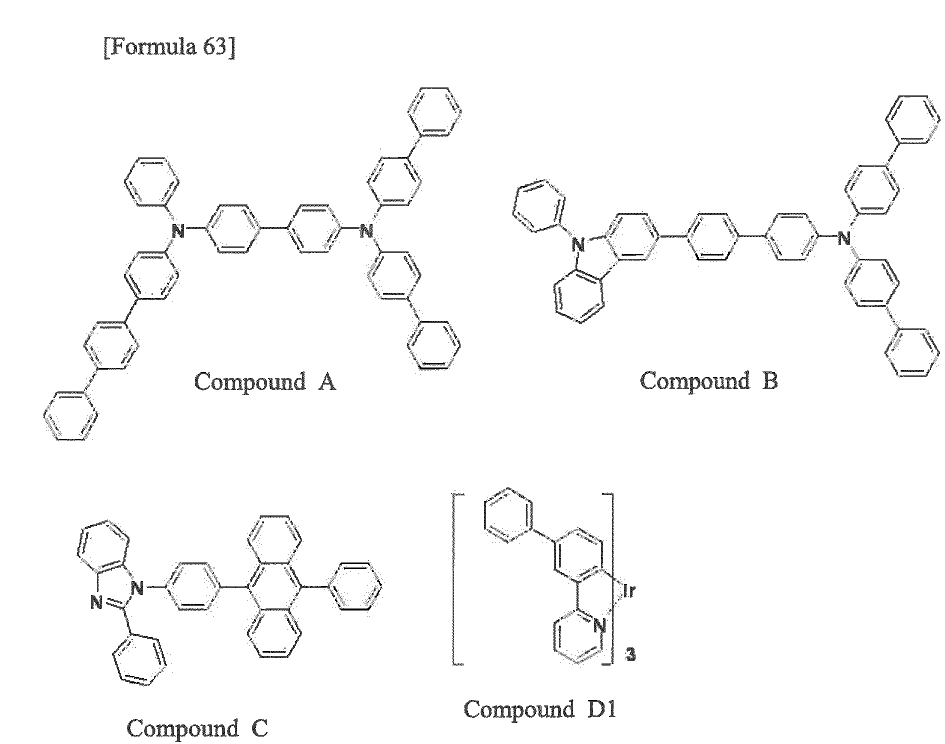

### Example 1

A glass substrate (size: 25 mm × 75 mm × 1.1 mm) having an ITO transparent electrode (manufactured by GEOMATEC Co., Ltd.) was ultrasonic-cleaned in isopropyl alcohol for five minutes, and then UV (Ultraviolet)/ozone-cleaned for 30 minutes.

After the glass substrate having the transparent electrode line was washed, the glass substrate was mounted on a substrate holder of a vacuum deposition apparatus, so that the compound A was vapor-deposited to form a 40-nm thick film of the compound A on a surface of the glass substrate where the transparent electrode line was provided so as to cover the transparent electrode. The film of the compound A was defined as a hole injecting layer.

The compound B was vapor-deposited on the film of the compound A to form a 20-nm thick film of the compound B. The film of the compound B was defined as a hole transporting layer.

On the hole transporting layer, the compound 1 obtained in Synthesis Example 1 was vapor-deposited to form a 40-nm thick emitting layer. The compound D1 (Ir(Ph-ppy)₃(facial body)) as a phosphorescent material was co-vapor-deposited with the compound 1. A concentration of the compound D1 was 20 mass%. The co-evaporation film serves as an emitting layer including the compound 1 as a phosphorescent host material and the compound D1 as a phosphorescent dopant material. Note that the compound D1 is a green-emitting material.

Following the film formation of the emitting layer, the compound C was vapor-deposited on the emitting layer to form a 30-nm thick film of the compound C. The film of the compound C was defined as an electron transporting layer.

Next, LiF was vapor-deposited on the film of the electron transporting layer at a film formation speed of 0.1 angstrom/min to form a 1-nm thick LiF film as an electron-injecting electrode (cathode).

A metal Al was further vapor-deposited on the LiF film to form an 80-nm thick metal cathode.

Thus, the organic EL device of Example 1 was prepared.

### Example 2

An organic EL device of Example 2 was prepared in the same manner as in the organic EL device of Example 1 except that the compound 2 was used in place of the compound 1 in the phosphorescent host material of the emitting layer of the organic EL device in Example 1.

### Example 3

An organic EL device of Example 3 was prepared in the same manner as in the organic EL device of Example 1 except that the compound 3 was used in place of the compound 1 in the phosphorescent host material of the emitting layer of the organic EL device in Example 1.

### Example 4

An organic EL device of Example 4 was prepared in the same manner as in the organic EL device of Example 1 except that the compound 4 was used in place of the compound 1 in the phosphorescent host material of the emitting layer of the organic EL device in Example 1.

### Evaluation of Organic EL Devices

The prepared organic EL devices were evaluated with respect to the luminous efficiency. The results are shown in Table 1.

### Measurement of Luminous Efficiency (Current Efficiency)

The prepared organic EL devices were driven by DC constant current (current density: 10 mA/cm²) at a room temperature to emit light, where spectral radiance spectra were measured by a spectro radiance meter (CS-1000: manufactured by Konica Minolta, Inc.). A luminous efficiency (unit: cd/A) was calculated from the obtained spectral radiance spectra. Note that values of voltage applied in measurement of the luminous efficiency are also shown in Table 1.

**Table 2**

| | Host Material | Voltage (V) | Luminous Efficiency (cd/A) |
|---|---|---|---|
| Example 1 | Compound 1 | 4.1 | 60 |
| Example 2 | Compound 2 | 4.3 | 63 |
| Example 3 | Compound 3 | 4.2 | 65 |
| Example 4 | Compound 4 | 4.1 | 62 |

As shown in Table 1, the organic EL devices according to Examples 1 to 4 exhibited an excellent luminous efficiency. The compounds 1 to 4 used in the phosphorescent host material for each of the organic EL devices in Examples 1 to 4 are the 4,3-bonded biscarbazole derivatives, triplet energy of each of which is relatively larger than that of the 3,3-bonded biscarbazole derivative. Accordingly, it is speculated that the triplet energy of the phosphorescent host material becomes larger than that of the compound D1 as the phosphorescent dopant material, thereby blocking transfer of triplet excitons from the phosphorescent dopant material to the phosphorescent host material to efficiently confine triplet excitons of the phosphorescent dopant material. Consequently, the luminous efficiency of the organic EL devices is believed to be improved by using the 4,3-bonded biscarbazole derivatives of the compounds 1 to 4 as the phosphorescent host material.

### INDUSTRIAL APPLICABILITY

A biscarbazole derivative of the invention is usable as an organic-EL-device material. An organic EL device using the biscarbazole derivative of the invention is usable as an emitting device in a display and an illuminator.

### EXPLANATION OF CODE(S)

1: organic EL device, 2: substrate, 3: anode, 4: cathode,
6: hole transporting layer, 7: emitting layer, 8: electron transporting layer,
10: organic compound layer

## Claims

1. A biscarbazole derivative represented by a formula (1) below, 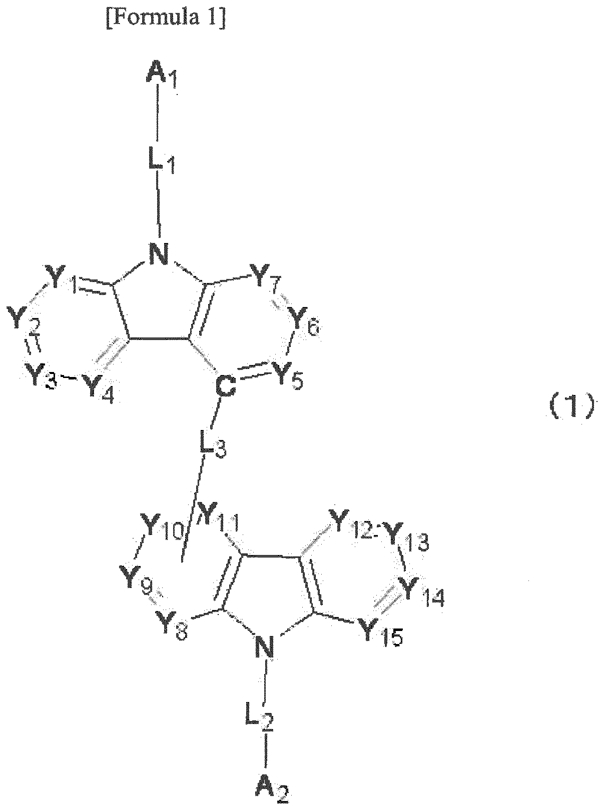 where: A₁ and A₂ represent a substituted or unsubstituted aromatic hydrocarbon group having 6 to 30 ring carbon atoms, or a substituted or unsubstituted aromatic heterocyclic group having 1 to 30 ring carbon atoms, with the proviso that at least one of A₁ and A₂ represents a substituted or unsubstituted aromatic heterocyclic group having 1 to 30 ring carbon atoms;
Y₁ to Y₄ each are a nitrogen atom or a carbon atom to be bonded to the following R, with the proviso that, when adjacent two of Y₁ to Y₄ are carbon atoms, a ring including the adjacent carbon atoms is optionally formed without the adjacent carbon atoms being bonded to R;
Y₅ to Y₇ are a nitrogen atom or a carbon atom to be bonded to the following R, with the proviso that, when adjacent two of Y₅ to Y₇ are carbon atoms, a ring including the adjacent carbon atoms is optionally formed without the adjacent carbon atoms being bonded to R;
one of Y₈ to Y₁₁ is a carbon atom bonded to L₃, and except for the one of Y₈ to Y₁₁ that is bonded to L₃, Y₈ to Y₁₁ are a nitrogen atom or a carbon atom to be bonded to the following R, with the proviso that, when adjacent two of Y₈ to Y₁₁ are carbon atoms, a ring including the adjacent carbon atoms is optionally formed without the adjacent carbon atoms being bonded to R;
Y₁₂ to Y₁₅ are a nitrogen atom or a carbon atom to be bonded to the following R, with the proviso that, when adjacent two of Y₁₂ to Y₁₅ are carbon atoms, a ring including the adjacent carbon atoms is optionally formed without the adjacent carbon atoms being bonded to R;
R each independently represents a hydrogen atom, a substituted or unsubstituted aromatic hydrocarbon group having 6 to 30 ring carbon atoms, a substituted or unsubstituted aromatic heterocyclic group having 1 to 30 ring carbon atoms, a substituted or unsubstituted linear, branched or cyclic alkyl group having 1 to 30 carbon atoms, a substituted or unsubstituted alkoxy group having 1 to 30 carbon atoms, a substituted or unsubstituted aryloxy group having 6 to 30 ring carbon atoms, a substituted or unsubstituted aralkyl group having 7 to 30 carbon atoms, a substituted or unsubstituted haloalkyl group having 1 to 30 carbon atoms, a substituted or unsubstituted haloalkoxy group having 1 to 30 carbon atoms, a substituted or unsubstituted trialkylsilyl group having 3 to 30 carbon atoms, a substituted or unsubstituted dialkylarylsilyl group having 8 to 40 carbon atoms, a substituted or unsubstituted alkyldiarylsilyl group having 13 to 50 carbon atoms, a substituted or unsubstituted triarylsilyl group having 18 to 60 carbon atoms, a halogen atom, a cyano group, a hydroxyl group, a nitro group, or a carboxy group; and
L₁ to L₃ represent a single bond or a divalent linking group, with the proviso that, when L₃ is a single bond and is bonded to Y₁₁, L₁ and L₂ are divalent linking groups.

2. The biscarbazole derivative according to claim 1, wherein
the biscarbazole derivative represented by the formula (1) is represented by the
following formula (2), (3) or (4),

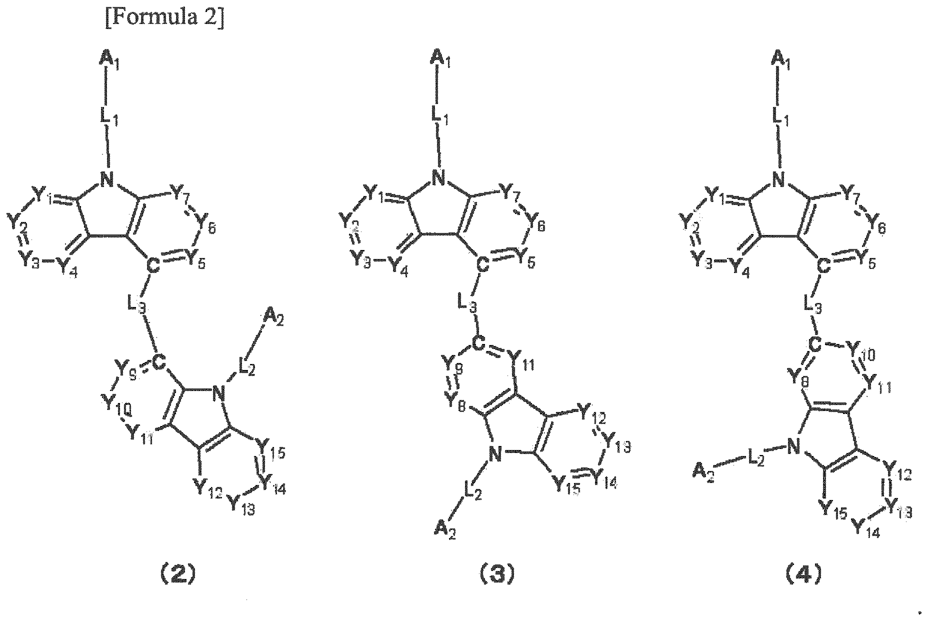

3. The biscarbazole derivative according to claim 1, wherein
the biscarbazole derivative represented by the formula (1) is represented by the following formula (3) or (4),

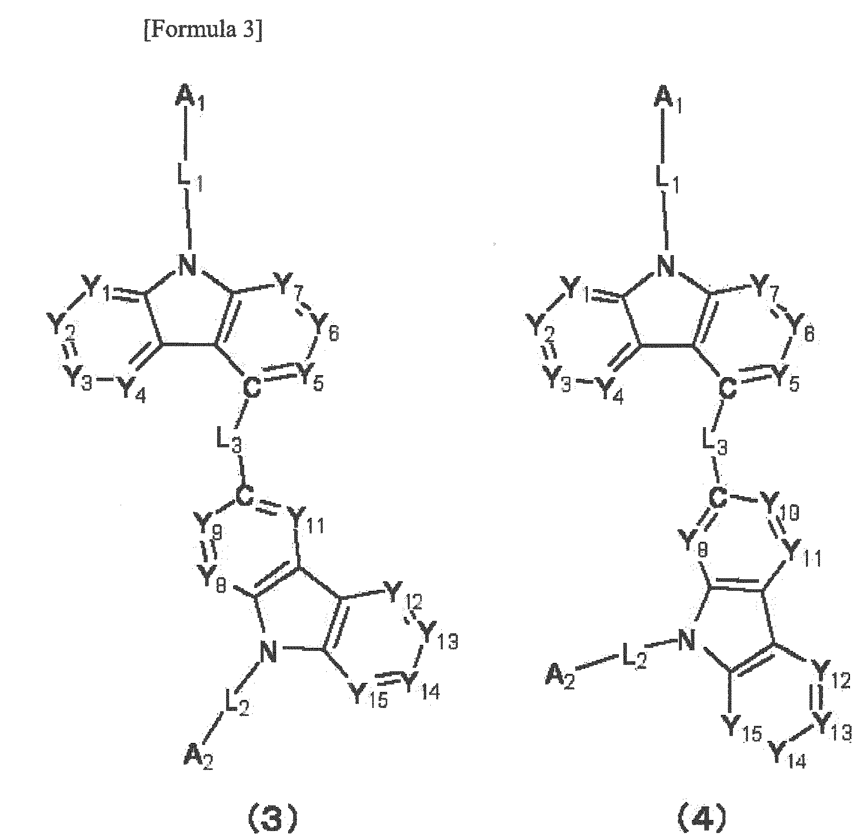

4. The biscarbazole derivative according to any one of claims 1 to 3, wherein
L₃ in the formula (1) is a single bond.

5. The biscarbazole derivative according to any one of claims 1 to 4, wherein
the aromatic heterocyclic group is a nitrogen-containing aromatic heterocyclic group.

6. The biscarbazole derivative according to claim 5, wherein
the nitrogen-containing aromatic heterocyclic group is a heterocyclic group having a pyrimidine skeleton or a heterocyclic group having a triazine skeleton.

7. The biscarbazole derivative according to any one of claims 1 to 6, wherein
at least one of L₁ and L₂ in the formula (1) is a divalent group derived from a substituted or unsubstituted aromatic hydrocarbon compound having 6 to 30 ring carbon atoms, or a divalent group derived from a substituted or unsubstituted aromatic heterocyclic compound having 1 to 30 ring carbon atoms.

8. An organic electroluminescence device comprising: a cathode; an anode; and an organic compound layer between the cathode and the anode, wherein
the organic compound layer comprises the biscarbazole derivative according to any one of claims 1 to 7.

9. The organic electroluminescence device according to Claim 8, wherein
the organic compound layer comprises a plurality of organic thin-film layers including an emitting layer, wherein
at least one of the plurality of organic thin-film layers comprises the biscarbazole derivative according to any one of claims 1 to 7.

10. The organic electroluminescence device according to claim 9, wherein
the emitting layer comprises the biscarbazole derivative according to any one of claims 1 to 7.

11. The organic electroluminescence device according to claim 9 or 10, wherein the emitting layer comprises a phosphorescent material.

12. The organic electroluminescence device according to claim 11, wherein
the phosphorescent material comprises an ortho-metalated complex of a metal atom selected from iridium (Ir), osmium (Os) and platinum (Pt).

13. The organic electroluminescence device according to any one of claims 10 to 12, wherein
the emitting layer further comprises an aromatic amine derivative.

14. The organic electroluminescence device according to any one of claims 8 to 13, wherein
the plurality of organic thin-film layers comprise a hole transporting layer, the emitting layer and an electron transporting layer.
